# EUROPEAN PATENT APPLICATION

(11) **EP 2 184 349 A1**
(43) Date of publication of application: **12.05.2010**
(21) Application number: 08792339.7
(22) Date of filing: 08.08.2008
(51) Int. Cl.: C12N 5/06, A61L 27/00, C12N 15/09

(54) **ISOLATED CELL MASS**

(30) Priority: 08.08.2007 JP 2007206823
(71) Applicant: Kyowa Hakko Kirin Co., Ltd., Tokyo 100-8185 (JP)
(72) Inventor: MIYAJI, Hiromasa, Machida-shi Tokyo 194-8533 (JP); IKKAKU, Masahiro, Machida-shi Tokyo 194-8533 (JP); SATO, Hidetaka, Machida-shi Tokyo 194-8533 (JP)
(74) Representative: Vossius & Partner
(86) International application number: PCT/JP2008/064282
(87) International publication number: WO 2009/020201

(57) **Abstract**

A cell having excellent migration ability and engraftment ability to tissues is required for the restoration and the like of damaged tissues making use of a hematopoietic stem cell transplantation or a stem cell transplantation. By the present invention, a cell population having excellent migration ability and engraftment ability to tissues, a pharmaceutical preparation comprising said cell and a transplantation method of said cell are provided. The cell of the present invention is useful for hematopoietic stem cell transplantation and restoration of damaged tissues using stem cell transplantation and the like.

## Description

### Technical Field

The present invention relates to a cell population having excellent migration ability and engraftment ability to tissues, a pharmaceutical preparation comprising said cells, and a transplantation method of said cells.

### Background Art

As the cell therapy most broadly carried out for the purpose of treating a damaged tissue, a transplantation which uses hematopoietic stem cells or hematopoietic progenitor cells has been carried out. Additionally, it has been reported that adult tissue stem cells and progenitor cells are present in various tissues and organs in the living body, and treatments using them have also been attempted.

The adult tissue stem cells are stem cells which are present in various living body tissues. It is considered that the cells have the self-renewal ability and differentiation ability and are contributing to the keeping of the homeostasis of tissues. The stem cells proliferate and are maintained in tissues. At the same time, the stem cells have only such a limited proliferation potency that the stem cells differentiate into progenitor cells in which the direction of differentiation is fixed and further differentiate into tissue cells having a specific function. For example, the hematopoietic stem cells are present mainly in bone marrow tissue in the case of adult and differentiate into a hematopoietic progenitor cells. Through its additional differentiation, the hematopoietic stem cells mature into all kinds of blood cells such as erythrocytes, leucocytes, thrombocytes and lymphocytes. In this manner, blood cells are maintained starting from their production by the hematopoietic stem cell.

The hematopoietic stem cell transplantation is a therapeutic method in which a strong treatment (pre-transplantation treatment) is carried out in order to increase the antitumor effect, using a large amount of antitumor agents or whole body radiation irradiation exceeding the maximum tolerance dose of the bone marrow, which leads the malignant tumor to its complete destruction together with the patient bone marrow, and then the hematopoietic capacity is recovered by transfusing hematopoietic stem cell derived from the allogenic donor or of the patient itself (autogenous) which had been cryopreserved in advance. The hematopoietic stem cell transplantation is classified into bone marrow transplantation, peripheral blood stem cell transplantation and cord blood transplantation depending on a collecting method of hematopoietic stem cells, and further classified into allograft and autograft depending on the origin of the hematopoietic stem cells. Transplantations such as the bone marrow transplantation, cord blood transplantation, peripheral blood stem cell transplantation and the like are carried out for the treatment of not only blood tumors such as leukemia, lymphoma and the like but also serious diseases such as solid cancer, autoimmune disease and the like.

Additionally, the treatment of diseased tissues by cell transplantation, using not only the hematopoietic stem cells and hematopoietic progenitor cells, but also bone marrow cells including mesenchymal stem cells and vascular endothelial progenitor cells, a population of cells including various stem cells and progenitor cells mobilized from bone marrow into peripheral blood by granulocyte-colony stimulating factor (G-CSF) and the like, cord blood-derived stem cells and progenitor cells and mesenchymal stem cells separated from bone marrow, fat tissue and the like has been attempted. A possibility of applying, by transplanting these cells to patients, to the treatment of many diseases such as graft versus host disease (GVHD), Crohn's disease, myocardial infarction, osteoporosis, cerebral infarction, hepatitis, pneumonia, diabetes mellitus, spinal cord injury, nephritis, bone fracture, lower limb ischemia and the like has been examined (cf., Non-patent References 1 to 12).

As described in the above, the hematopoietic stem cell transplantation is classified into bone marrow transplantation, peripheral blood transplantation and cord blood transplantation depending on the difference in donor cells. The number of cases of transplantation has been increasing in recent years particularly on the cord blood transplantation, in view of its various advantages in that cord blood can be easily used without a burden of its donor since it is originally a waste, that cord blood is possible to be transplanted even when the coinciding degree of histocompatibility antigen is low and that it is possible to carry out emergency measure due to preparation of cord blood bank (cf., Non-patent Reference 13). However, since the number of cells in cord blood is small, the cord blood transplantation has a disadvantage in that its object to be transplanted is mainly children and it is unsuitable for adults (cf., Non-patent Reference 14). Additionally, the cord blood transplantation has a poor transplantation result in comparison with the bone marrow transplantation and peripheral blood transplantation by the reasons of insufficient engraftment after transplantation and delay of the restoration of platelets and neutrophil (cf., Non-patent References 13 and 15). Regarding the clinical result of hematopoietic stem cell transplantation of bone marrow and cord blood, it is known that the survival rate is high and the restoration of platelets and neutrophil is quick, when the number of hematopoietic stem cells (CD 34 positive cells) to be transplanted is large (cf., Non-patent References 16 to 18). Thus, it is necessary to keep a large number of hematopoietic stem cells and effect efficient engraftment of the hematopoietic stem cells in the field of transplantation, and it is expected that techniques and products which can resolve such problems will improve the transplantation result of hematopoietic stem cells and contribute to the lifesaving, reduction of medical expenses and quality of life of patients.

In the mature body, the hematopoietic stem cells maintain the hematopoietic system by repeating its proliferation and differentiation in the bone marrow tissue. The transplanted hematopoietic stem cells find its way to the bone marrow at last via peripheral blood and engraft to the inside of bone marrow. Such a way of migration to a certain tissue and engraftment thereto is called homing. Interaction of hematopoietic stem cells and bone marrow blood vessel is important for the homing of the hematopoietic stem cells to bone marrow. The interaction occurs at the step in which the hematopoietic stem cells cause rolling and adhesion on the bone marrow vascular endothelial cells, and as a result of this interaction, the hematopoietic stem cells pass through the vascular endothelial cells and migrate to the inside of the marrow. The rolling and adhesion are controlled by the adhesion molecules on the hematopoietic stem cells and bone marrow vascular endothelial cells. It is known that E-selectin and P-selectin are important as the molecules expressing in the bone marrow vascular endothelial cells which relate to engraftment of hematopoietic stem cells (cf., Non-patent Reference 19). On the other hand, two or more adhesion molecules expressing in the hematopoietic stem cells are present as glycoproteins. P-Selectin glycoprotein ligand-1 (PSGL-1) as a ligand of P-selectin (cf., Non-patent References 20 to 23) is known. As a ligand of E-selectin, α-4 integrin (cf., Non-patent Reference 24), CD 44 (cf., Non-patent Reference 25) and E-selectin ligand-1 (ESL-1) (cf., Non-patent Reference 26) are known. Sialyl Lewis X sugar chains formed by α 2,3-sialylation and α 1,3-fucosylation of sugar chains on these core proteins function as ligands of selectin.

The enzyme which catalyzes the α 1,3-fucosylation is called α 1,3-fucosyltransferase (FT), and 6 kinds thereof (FT 3, FT 4, FT 5, FT 6, FT 7 and FT 9) are known in human. Among them, expression of FT 4, FT 7 and FT 9 in blood cells has been confirmed. Each FT has different enzyme reactivity, and sialyl Lewis X sugar chain alone is formed by the fucosylation by FT 7, but in addition to sialyl Lewis X sugar chain, Lewis X sugar chain is also formed by the fucosylation by FT 6. Although the Lewis X sugar chain is expressed in blood cells such as monocyte, granulocyte and the like, it does not have a function as a selectin ligand. The expression region, expression-controlling mechanism, reaction specificity and the like have not so far been sufficiently revealed on the above-mentioned 6 kinds of FT (cf., Non-patent Reference 27).

A method for increasing the amount of selectin ligands by treating the hematopoietic stem cells with FT *ex vivo* and adding sialyl Lewis X sugar chains has been reported. Xia *et al.* confirmed that sialyl Lewis X sugar chains are increased and binding ability to E-selectin and P-selectin is increased when a cell line is treated with FT 6 (cf., Non-patent Reference 28). Also, Hindalgo *et al.* suggested a possibility that posttranslational modification of PSGL-1 is important since cord blood-derived CD 34-positive cells express PSGL-1 but do not have the binding ability to P-selectin (cf., Non-patent Reference 29). Additionally, it is known that the cord blood-derived CD 34-positive cells treated with FT 6 have increased sialyl Lewis X sugar chains, increased binding ability to E-selectin and P-selectin, reinforced interaction of the bone marrow vascular endothelium and said CD 34-positive cell (rolling and arrest) in transplantation (cf., Patent Reference 1). However, it was found that the homing efficiency of said cells to bone marrow is not improved. It has been suggested that although its interaction with the vascular endothelial cells is improved by the addition of sialyl Lewis X sugar chain and the increase of its binding ability to E-selectin and P-selectin by FT 6, there is no effect at the homing step thereafter for migration and engraftment to the inside of bone marrow (cf., Non-patent Reference 30). On the other hand, Xia *et al.* reported that the ratio of human blood cells in bone marrow increased about two times after 6 weeks of transplantation in a transplantation test of the mononuclear cells derived from human cord blood which were similarly treated with FT6 (cf., Non-patent Reference 31 and Patent Reference 2).

Additionally, a method was reported for increasing the amount of selectin ligand by adding a sialyl Lewis X sugar chain through the *ex vivo* FT6 treatment of a mesenchymal stem cell. In a transplantation test of the FT6-treated mesenchymal stem cell, increase in the number of mesenchymal stem cells migrated to the bone marrow was observed (cf., Non-patent Reference 32).

Also, a method was reported for increasing the amount of selectin ligand by adding a sialyl Lewis X sugar chain through the *ex vivo* FT6 treatment of a neural stem cell (cf., Patent Reference 3).

Although there are such reports described above, nothing is known about the influence of the kinds of FT and difference in specificity upon the homing ability of hematopoietic stem cells. Also, completely nothing is known about the influence of the expression of Lewis X sugar chain on the migration ability and engraftment ability of stem cells to the bone marrow tissue and the like. Additionally, nothing is known about the influence of the FT 7 as a kind of α 1,3-fucosyltransferase on the migration ability and engraftment ability of the hematopoietic stem cells , mesenchymal stem cells, neural stem cells and the like to the tissues.
Patent Reference 1: WO 2005/017115
Patent Reference 2: WO 2004/094619
Patent Reference 3: US2008/0044383
Non-patent Reference 1: Saishin Igaku, Gendai Iryo no Saizensen, Extra Issue, March, 2003, Saishin Igaku Company
Non-patent Reference 2: Igaku no Ayumi, vol. 217, No. 5, 2006, Ishiyaku Pub. Inc.
Non-patent Reference 3: Current Opinion in Investigational Drugs, 7, 473 - 81 (2006)
Non-patent Reference 4: Stem Cells, 24, 2292 - 2298 (2006)
Non-patent Reference 5: J. Cell. Physiol., 211, 27 - 35 (2007)
Non-patent Reference 6: Experimental Neurology, 199, 56 - 66 (2006)
Non-patent Reference 7: Cellular and Molecular Neurology, 26, 1167 - 1180 (2006)
Non-patent Reference 8: Proc. Natl. Acad. Sci. USA, 103, 17438 - 17443 (2006)
Non-patent Reference 9: FASEB J., 19, 992 - 994 (2005)
Non-patent Reference 10: American Heart J., 153, el - 8 (2007)
Non-patent Reference 11: J. Clin. Invest., 114, 330 - 338 (2004)
Non-patent Reference 12: Proc. Am. Thorac. Soc., 5, 323-327 (2008)
Non-patent Reference 13: Blood, 97, 2962 - 2971 (2001)
Non-patent Reference 14: N. Eng. J. Med., 339, 1565 - 1577 (1998)
Non-patent Reference 15: Blood, 97, 2957 - 2961 (2001)
Non-patent Reference 16: N. Eng. J. Med., 344, 1815 - 1822 (2001)
Non-patent Reference 17: Blood, 100, 1611 - 1618 (2002)
Non-patent Reference 18: British J. Haematology, 124, 769 - 776 (2004)
Non-patent Reference 19: Proc. Natl. Acad. Sci. USA, 95, 14423 - 14428 (1998)
Non-patent Reference 20: J. Cell Biol., 118, 445 - 456 (1992)
Non-patent Reference 21: J. Exp. Med., 191, 1413 - 1432 (2000)
Non-patent Reference 22: Blood, 85, 3466 - 3477 (1995)
Non-patent Reference 23: Exp. Haematol., 24, 1494 - 1500 (1996)
Non-patent Reference 24: Blood, 102, 2060 - 2067 (2003)
Non-patent Reference 25: J. Cell Biol., 153, 1277 - 1286 (2001)
Non-patent Reference 26: J. Biol. Chem., 276, 31602 - 31612 (2001)
Non-patent Reference 27: Lowe, J.B., D. Vestweber, ed (Harwood Academic Publishers), pp. 143 - 177 (1996)
Non-patent Reference 28: Blood, 100, 4485 - 4494 (2002)
Non-patent Reference 29: J. Clin. Invest., 110, 559 - 569 (2002)
Non-patent Reference 30: Blood, 105, 567 - 575 (2005)
Non-patent Reference 31: Blood, 104, 3091 - 3096 (2004)
Non-patent Reference 32: Nature Medicine, 14, 181-187 (2008)

### Disclosure of the Invention

### Problems to be Solved by the Invention

In case where a cell population with excellent migration and engraftment of transplanted cells to the bone marrow and damaged tissues, in the transplantation of various cells such as hematopoietic stem cells, hematopoietic progenitor cells and the like, can be obtained, therapeutic effect with less cells can be expected, and not only the improvement of therapeutic effect but also the resolution of the aforementioned problems can be expected.

The object of the invention is to provide a cell population having excellent migration ability and engraftment ability to tissues, a pharmaceutical preparation comprising said cell population, and a transplantation method of said cell population.

### Means for Solving the Problems

The present invention relates to the following (1) to (49).
(1) An isolated cell population, wherein a sialyl Lewis X sugar chain is expressed and a Lewis X sugar chain is not substantially expressed.
(2) The cell population according to (1), which is obtained by a cell fractionation treatment.
(3) The cell population according to (1) or (2), which is obtained by treating a cell with an α 1,3-fucosyltransferase in the presence of a fucose donor.
(4) The cell population according to (3), wherein the α 1,3-fucosyltransferase is α 1,3-fucosyltransferase 7.
(5) The cell population according to (3), wherein the α 1,3-fucosyltransferase is a polypeptide consisting of the amino acid sequence of SEQ ID NO: 1.
(6) The cell population according to (3), wherein the α 1,3-fucosyltransferase consists of an amino acid sequence having a homology of 60% or more with a polypeptide consisting of the amino acid sequence of SEQ ID NO:1 and also has α 1,3-fucosyltransferase activity.
(7) The cell population according to (3), wherein the α 1,3-fucosyltransferase consists of an amino acid sequence in which at least one amino acid in the amino acid sequence of SEQ ID NO:1 is substituted, deleted or added, and also has α 1,3-fucosyltransferase activity.
(8) The cell population according to (3), wherein the α 1,3-fucosyltransferase is a polypeptide encoded by a DNA consisting of the nucleotide sequence of SEQ ID NO:2.
(9) The cell population according to (3), wherein the α 1,3-fucosyltransferase is a polypeptide encoded by a DNA which hybridizes with a DNA consisting of a nucleotide sequence complementary to the nucleotide sequence of SEQ ID NO:2 under a stringent condition, and also is a polypeptide having α 1,3-fucosyltransferase activity.
(10) The cell population according to any one of the (1) to (9), wherein the cell is a stem cell or a progenitor cell.
(11) The cell population according to (10), wherein the stem cell or progenitor cell is a hematopoietic stem cell or a hematopoietic progenitor cell.
(12) The cell population according to (11), wherein the hematopoietic stem cell or hematopoietic progenitor cell is a CD 34-positive hematopoietic stem cell or a CD 34-positive hematopoietic progenitor cell.
(13) The cell population according to (12), wherein the CD 34-positive hematopoietic stem cell is a CD 38-negative hematopoietic stem cell.
(14) The cell population according to (11), wherein the hematopoietic stem cell or hematopoietic progenitor cell is a CD 34-negative and Lineage-negative hematopoietic stem cell or CD 34-negative and Lineage-negative hematopoietic progenitor cell.
(15) The cell population according to (10), wherein the stem cell is a mesenchymal stem cell.
(16) The cell population according to (10), wherein the stem cell or progenitor cell is a neural stem cell or neural progenitor cell.
(17) The cell population according to (16), wherein the neural stem cell or neural progenitor cell is Nestin-positive neural stem cell or Nestin-positive neural progenitor cell.
(18) The cell population according to (16), wherein the neural stem cell or neural progenitor cell is Sox2-positive neural stem cell or Sox2- positive neural progenitor cell.
(19) The cell population according to (16), wherein the neural stem cell or neural progenitor cell is Musashi1-positive neural stem cell or Musashi 1-positive neural progenitor cell.
(20) The cell population according to (16), wherein the neural stem cell or neural progenitor cell is CD34-positive neural stem cell or CD34-positive neural progenitor cell.
(21) The cell population according to (16), wherein the neural stem cell or neural progenitor cell is CD133-positive neural stem cell or CD133-positive neural progenitor cell.
(22) The cell population according to any one of (1) to (21), wherein the cell is a human-derived cell.
(23) The cell population according to any one of the (1) to (22), wherein the cell is a cell derived from a tissue selected from the group consisting of bone marrow, spleen, cord blood, peripheral blood, fat tissue and neural tissue.
(24) A pharmaceutical preparation, which comprises the cell population according to any one of (1) to (23).
(25) A method for transplanting a cell population, which comprises administering the cell population according to any one of (1) to (23) to a living body.
(26) A method for producing a cell expressing sialyl Lewis X sugar chain but not substantially expressing Lewis X sugar chain, which comprises obtaining the cell by a fractionation treatment.
(27) A method for producing a cell expressing sialyl Lewis X sugar chain but not substantially expressing Lewis X sugar chain, which comprises obtaining the cell by treating with an α 1,3-fucosyltransferase in the presence of a fucose donor.
(28) The production method according to (27), wherein the α 1,3-fucosyltransferase is α 1,3-fucosyltransferase 7.
(29) The production method according to (27), wherein the α 1,3-fucosyltransferase is a polypeptide consisting of the amino acid sequence of SEQ ID NO:1.
(30) The production method according to (27), wherein the α 1,3-fucosyltransferase is a polypeptide consisting of an amino acid sequence having a 60% or more of homology with the polypeptide consisting of the amino acid sequence of SEQ ID NO:1 and also having the α 1,3-fucosyltransferase activity.
(31) The production method according to (27), wherein the α 1,3-fucosyltransferase is a polypeptide consisting of an amino acid sequence in which at least one amino acid in the amino acid sequence of SEQ ID NO:1 is substituted, deleted or added and also having the α 1,3-fucosyltransferase activity.
(32) The production method according to (27), wherein the α 1,3-fucosyltransferase is a polypeptide encoded by a DNA consisting of the nucleotide sequence of SEQ ID NO:2.
(33) The production method according to (27), wherein the α 1,3-fucosyltransferase is a polypeptide encoded by a DNA which hybridizes with a DNA consisting of a nucleotide sequence complementary to the nucleotide sequence of SEQ ID NO:2 under a stringent condition, and also having the α 1,3-fucosyltransferase activity.
(34) The production method according to (26) to (33), wherein the cell is a stem cell or a progenitor cell.
(35) The production method according to (34), wherein the stem cell or progenitor cell is a hematopoietic stem cell or a hematopoietic progenitor cell.
(36) The production method according to (35), wherein the hematopoietic stem cell or hematopoietic progenitor cell is a CD 34-positive hematopoietic stem cell or a CD 34-positive hematopoietic progenitor cell.
(37) The production method according to (36), wherein the CD 34-positive hematopoietic stem cell is a CD 38-negative hematopoietic stem cell.
(38) The production method according to (35), wherein the hematopoietic stem cell or hematopoietic progenitor cell is a CD 34-negative and Lineage-negative hematopoietic stem cell or CD 34-negative and Lineage-negative hematopoietic progenitor cell.
(39) The production method according to (34), wherein the stem cell is a mesenchymal stem cell.
(40) The production method according to (34), wherein the stem cell or progenitor cell is a neural stem cell or neural progenitor cell.
(41) The production method according to (40), wherein the neural stem cell or neural progenitor cell is Nestin-positive neural stem cell or Nestin-positive neural progenitor cell.
(42) The production method according to (40), wherein the neural stem cell or neural progenitor cell is Sox2-positive neural stem cell or Sox2-positive neural progenitor cell.
(43) The production method according to (40), wherein the neural stem cell or neural progenitor cell is Musashi1-positive neural stem cell or Musashi 1-positive neural progenitor cell.
(44) The production method according to (40), wherein the neural stem cell or neural progenitor cell is CD34-positive neural stem cell or CD34-positive neural progenitor cell.
(45) The production method according to (40), wherein the neural stem cell or neural progenitor cell is CD133-positive neural stem cell or CD133-positive neural progenitor cell.
(46) The production method according to any one of (26) to (45), wherein the cell is a human-derived cell.
(47) The production method according to any one of (26) to (46), wherein the cell is a cell derived from a tissue selected from the group consisting of bone marrow, spleen, cord blood, peripheral blood, fat tissue and neural tissue.
(48) A pharmaceutical preparation, which comprises a cell obtained by the production method according to any one of (26) to (47).
(49) A method for transplanting a cell, which comprises administering a cell obtained by the production method according to any one of (26) to (47) to a living body.

### EFFECT OF THE INVENTION

According to the present invention, a cell population having excellent migration ability and engraftment ability to tissues, a pharmaceutical preparation comprising said cell population and a transplantation method of said cell population are provided. The cell population of the present invention is useful for restoration of damaged tissues and the like.

### BRIEF DESCRIPTION OF THE DRAWINGS

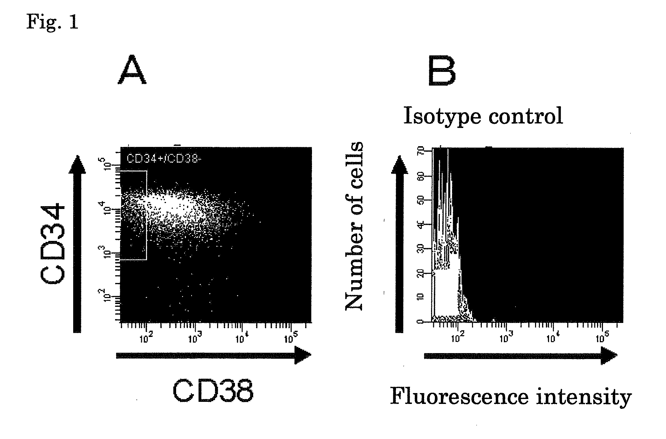

[Fig. 1] Fig. 1 is a figure showing a result of the FACS analysis of CD 34-positive cells separated from cord blood mononuclear cells. Purity of the separated CD 34 cells is 95% or more. A group of the CD 34-positive CD 38-negative cells, as human hematopoietic stem cells, was gated in A of the left side drawing and analyzed.

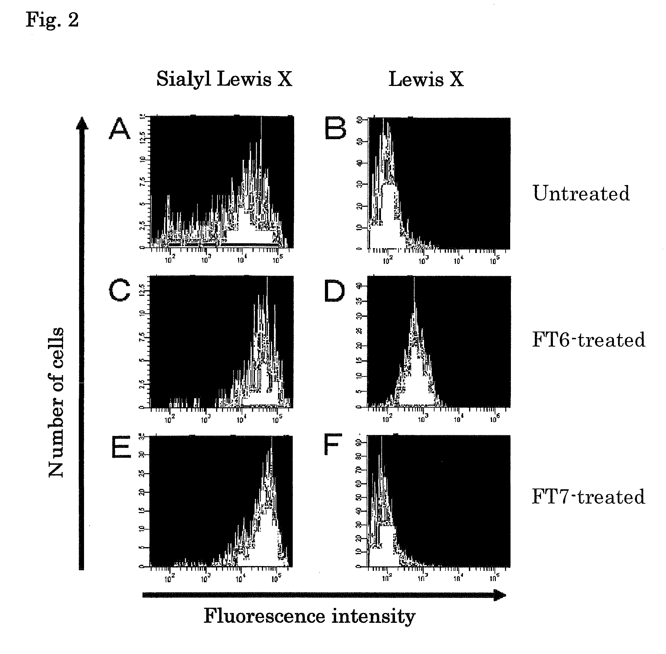

[Fig. 2] Fig. 2 is a figure showing a result of comparing expressed quantities of sialyl Lewis X sugar chain and Lewis X sugar chain of human hematopoietic stem cells (CD 34-positive CD 38-negative cells) treated with FT 6 or FT 7 to those of untreated cells. An expressed quantity of sialyl Lewis X sugar chain is shown in A, C and E, and an expressed quantity of Lewis X sugar chain is shown in B, D and F.

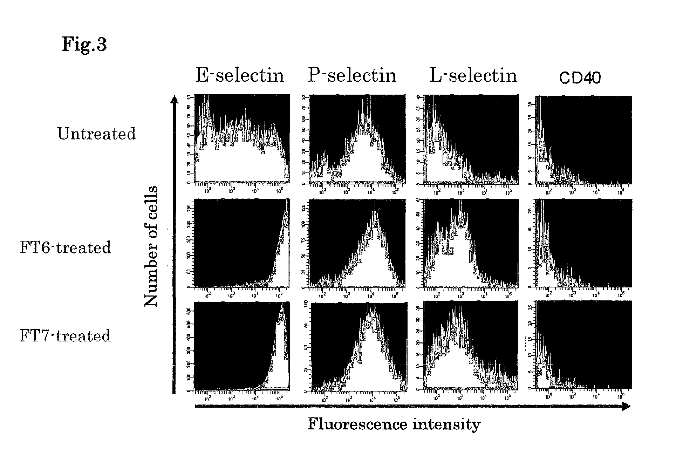

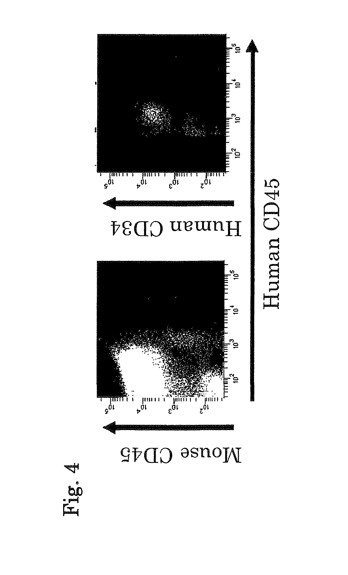

[Fig. 3] Fig. 3 is a figure showing a result of comparing selectin binding ability of human hematopoietic stem cells (CD 34-positive CD 38-negative cells) treated with FT 6 or FT 7 to that of untreated cells. As a negative control, a result of the analysis of cells treated with CD 40-Fc chimeric protein is shown. [Fig. 4] Fig. 4 is a figure showing a result of comparing bone marrow migration ability of human hematopoietic stem cells (CD 34-positive CD 38-negative cells) treated with FT 6 or FT 7 to that of untreated cells. An example of the FACS analysis of human hematopoietic stem cells (CD 45-positive CD 34-positive cells) migrated to the bone marrow of NOD/SCID mouse after 12 to 18 hours of the transplantation is shown in the right side drawing.

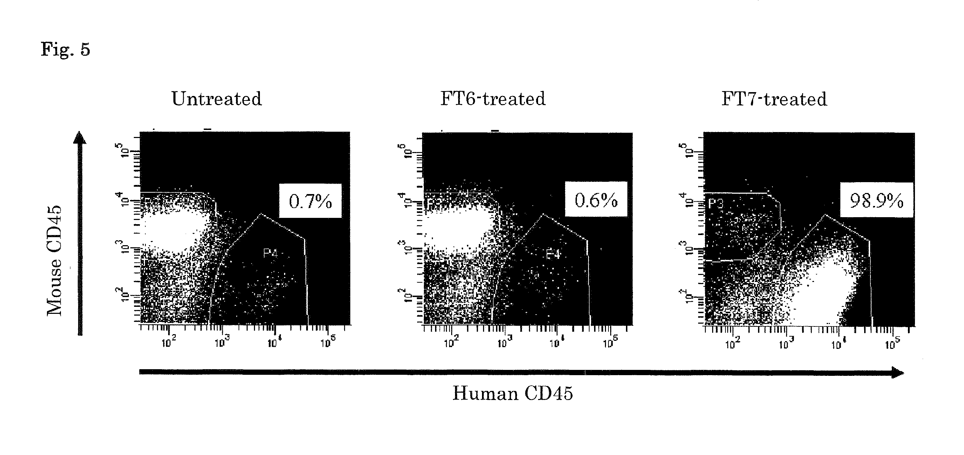

[Fig. 5] Fig. 5 is a figure showing a result of comparing engraftment ratio of human blood cells in bone marrow of human hematopoietic stem cells (CD 34-positive CD 38-negative cells) treated with FT 6 or FT 7 to that of untreated cells. It shows human blood cells engrafted to the bone marrow of NOD/SCID mouse after 8 to 9 weeks of the transplantation. The human CD 45-positive cells shown by a gate indicate human blood cells, and the mouse CD 45-positive cells indicate mouse blood cells.

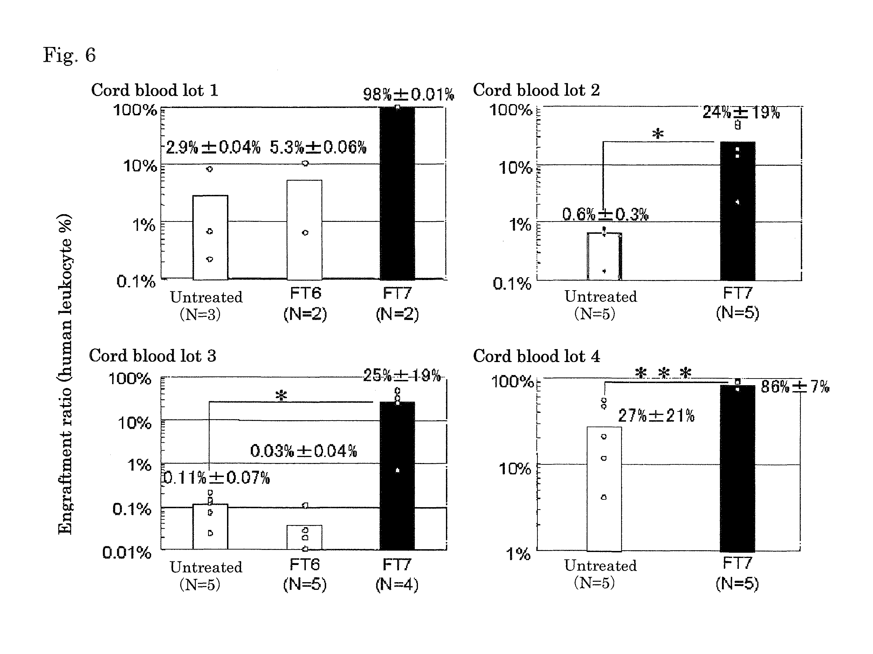

[Fig. 6] Fig. 6 is a figure showing a result of comparing engraftment ratio of human blood cells in bone marrow of human hematopoietic stem cells (CD 34-positive CD 38-negative cells) treated with FT 6 or FT 7 to that of untreated cells. It shows human blood cells engrafted to the bone marrow of NOD/SCID mouse after 8 to 9 weeks of the transplantation. The axis of ordinate is a logarithmic expression of the engraftment ratio of human blood cells (ratio of human blood cells). The value of the axis of ordinate shows average value ± SD of the ratio (%) of human blood cells.

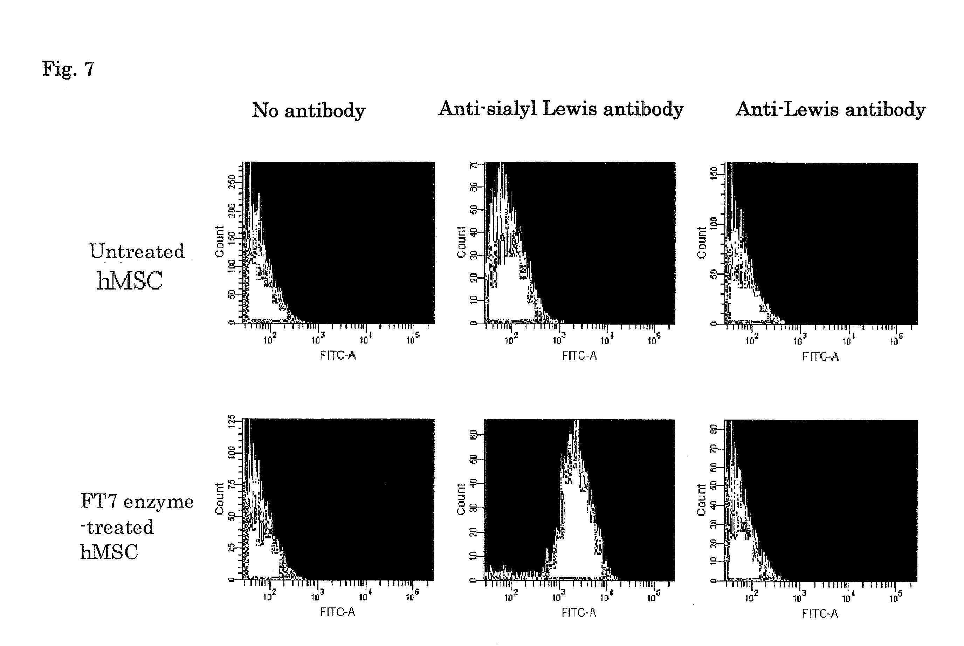

[Fig. 7] Fig. 7 shows a result of the analysis of enzyme-untreated or FT 7-treated human mesenchymal stem cells (hMSC) by flow cytometry. The "no antibody" shows a result of the analysis of hMSC which was not treated with antibody, and the "anti-sialyl Lewis X antibody" shows that of hMSC which was treated with anti-sialyl Lewis X sugar chain antibody and then treated with FITC-labeled anti-mouse IgG antibody as the secondary antibody, and the "anti-Lewis X antibody" shows that of hMSC which was treated with FITC-labeled anti-human CD 15 antibody. The axis of ordinate shows the number of cells and the axis of abscissa shows fluorescence intensity.

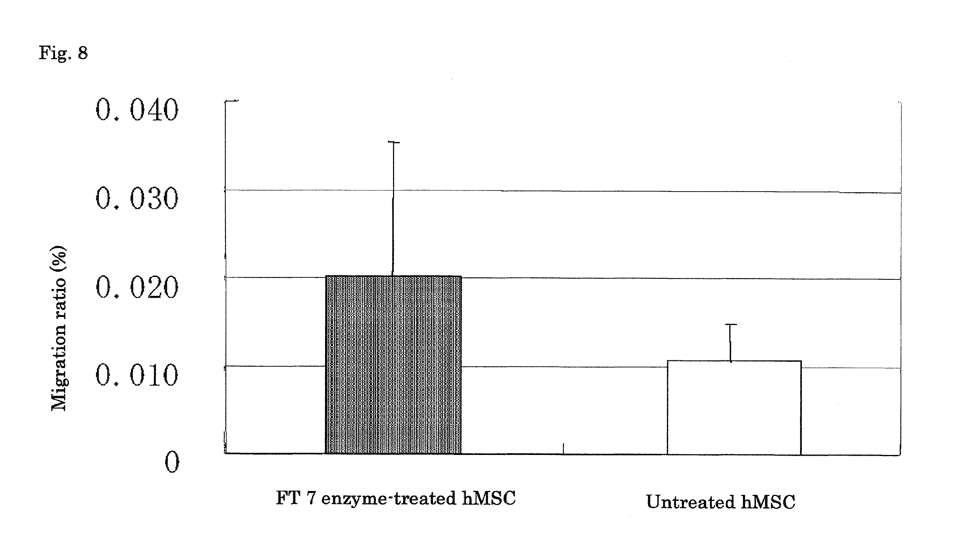

[Fig. 8] Fig. 8 is a result of transplanting FT 7 enzyme-treated hMSC or untreated hMSC (negative control) to a mouse carbon tetrachloride hepatopathy model at a dose of 1 × 10⁵ cells (n = 5 for each group); extracting the liver after 24 hours and treating it with collagenase; treating the thus obtained cells with biotin-labeled Mouse Lineage Panel; subsequently treating them with PerCP-labeled streptoavidin and PE-labeled anti-human CD 90 antibody; and then analyzing the resulting cells by flow cytometry. The number of PE-positive cells per about 1.5 × 10⁶ liver cells was calculated as the migration ratio (%) of the transplanted hMSC to the liver. The value of the axis of ordinate shows average ± SD of the migration ratio in each individual.

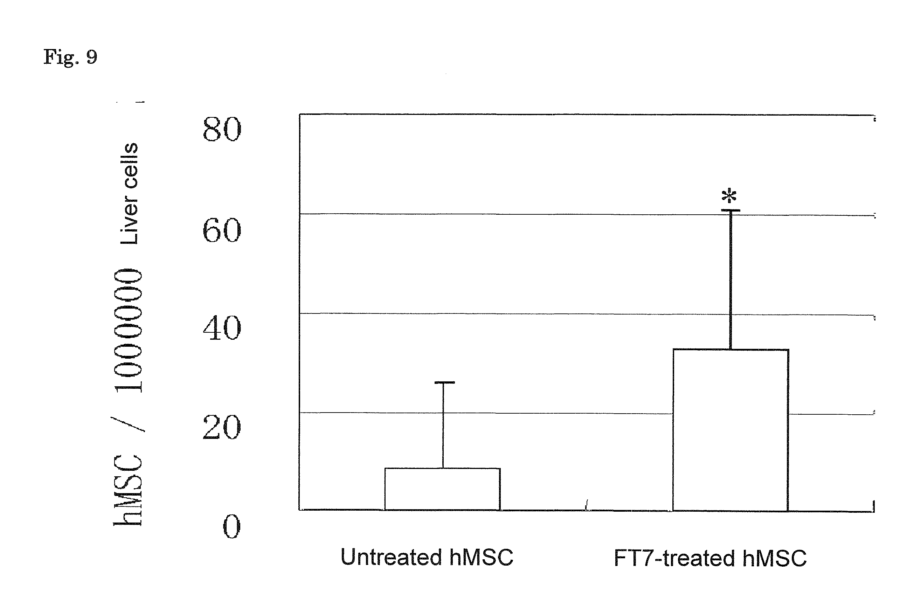

[Fig. 9] Fig. 9 is a result obtained by respectively transplanting 5 × 10⁵ cells of an FT7 enzyme-treated hMSC or untreated hMSC (negative control) to a mouse carbon tetrachloride hepatopathy model (n = 6 for each group); extracting the liver after 18 hours and treating it with collagenase; and analyzing the thus obtained cells by a flow cytometry after treating them with a PE-Cy7-labeled streptoavidin, an FITC-labeled anti-mouse CD45 antibody, an FITC-labeled anti-mouse Ter 119 antibody and a PE-labeled anti-human CD90 antibody. The number of PE-positive cells per 1 × 10⁶ liver cells was calculated as the number of hMSC migrated to the liver. The value on the axis of ordinate shows average ± SD of the number of migrated cells in each individual.

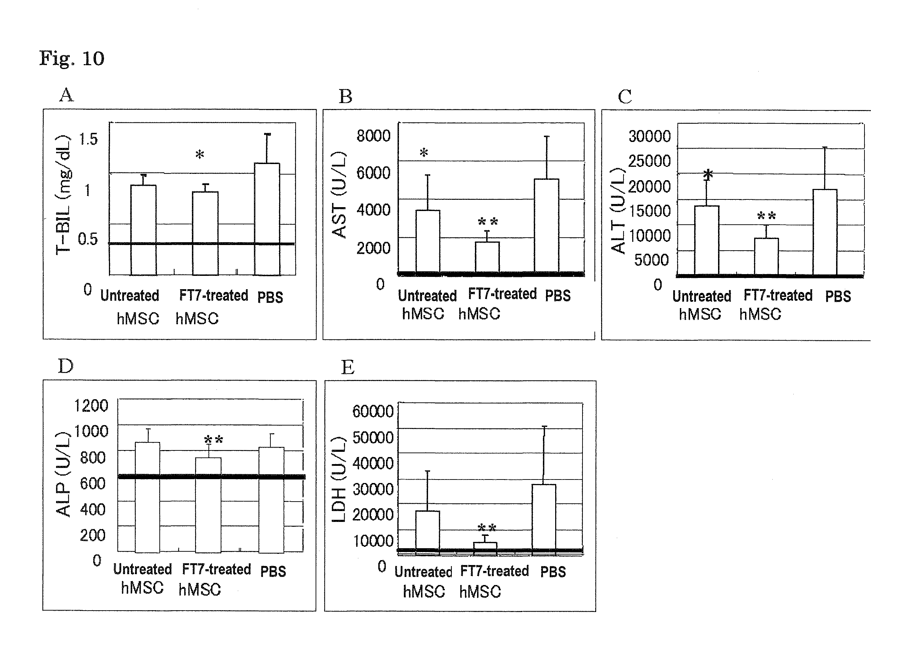

[Fig. 10] Fig. 10 is a result obtained by respectively administering 5 × 0⁵ cells of an FT 7 enzyme-treated hMSC or untreated hMSC (negative control), or a solvent (PBS), to a mouse carbon tetrachloride hepatopathy model (n = 6 for each group), and measuring blood biochemical parameters [(A) Total-Bilirubin, (B) AST, (C) ALT, (D) ALP, (E) LDH] after 18 hours by Fuji Dry Chem. The value on the axis of ordinate shows average ± SD of the values in each individual.

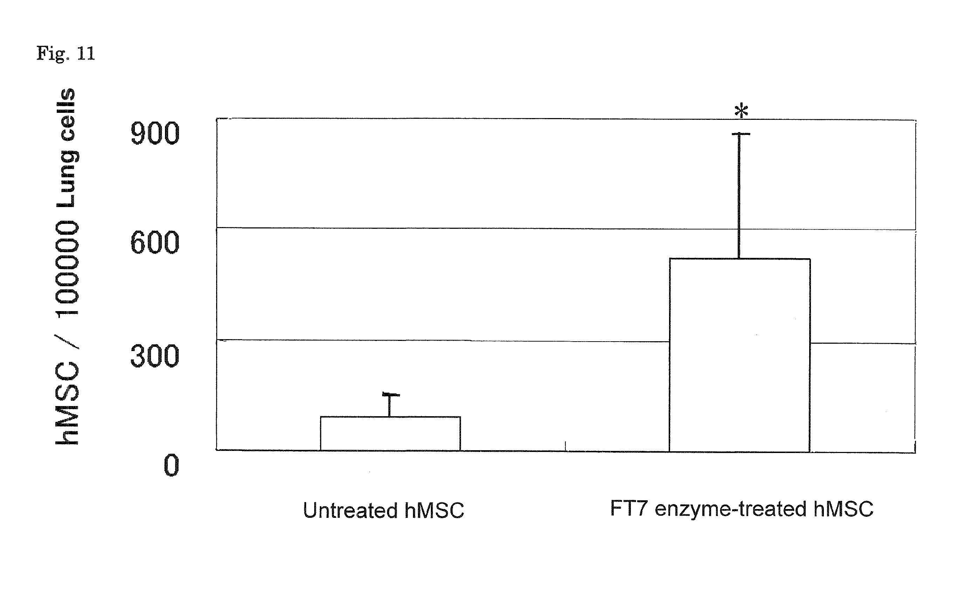

[Fig. 11] Fig. 11 is a result obtained by respectively transplanting 5 × 10⁵ cells of an FT7 enzyme-treated hMSC or untreated hMSC (negative control) to a mouse carbon tetrachloride lung disorder model (n = 5 for each group); extracting the lungs 18 hours thereafter and treating them with collagenase-elastase; and analyzing the thus obtained cells by a flow cytometry after treating them with a PE-Cy7-labeled streptoavidin, an FITC-labeled anti-mouse CD45 antibody, an FITC-labeled anti-mouse Ter 119 antibody and a PE-labeled anti-human CD90 antibody. The number of PE-positive cells per 1 × 10⁵ lung cells was calculated as the number of hMSC migrated to the lungs. The value on the axis of ordinate shows average ± SD of the number of migrated cells in each individual.

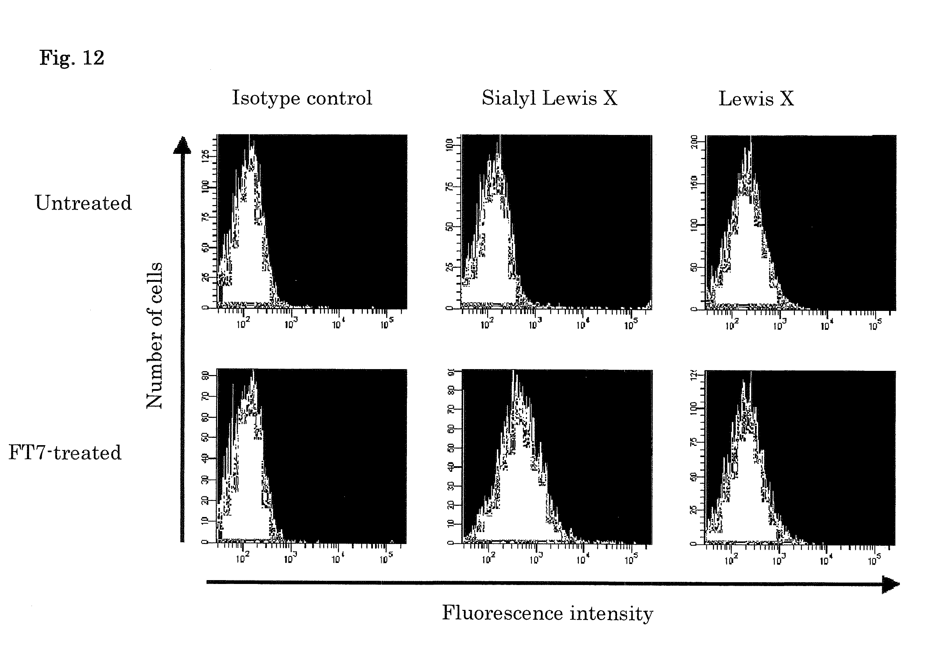

[Fig. 12] Fig. 12 is a a result in which the expressed amounts of sialyl Lewis X sugar chain and Lewis X sugar chain with treating neural stem cells or neural progenitor cells with FT7, were compared with the case of untreated cells.

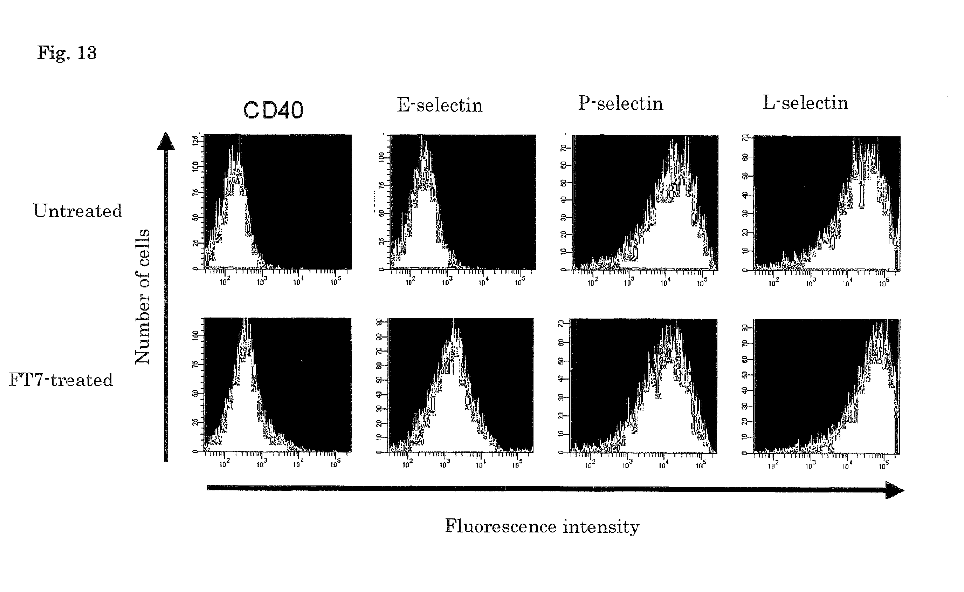

[Fig. 13] Fig. 13 is a graph showing a result in which the ability to bind to various species of selectin with treating neural stem cells or neural progenitor cells with FT7, was compared with the case of untreated cells.

### Best Mode for Carrying Out the Invention

### 1. Cell of the present invention

The cell population of the present invention can be prepared by isolating from cells comprising a cell which expresses sialyl Lewis X sugar chains but substantially does not express Lewis X sugar chains. Although the cells comprising the cell population of the present invention may be any cells as long as they comprise the cell population of the present invention, it is desirable that stem cells or progenitor cells are contained therein.

In this connection, the term "substantially does not express Lewis X sugar chains" means that the Lewis X sugar chain is negative or equivalent to negative and its degree is significantly low in comparison with the expression of sialyl Lewis X sugar chain. It also means that expression of Lewis X sugar chain is the same level of expression when Lewis X sugar chain is not artificially expressed.

Although the cells comprising the cell population of the present invention may be any animal-derived cells, human-derived cells, non-human primates-derived cells, cattle-derived cells; horse-derived cells, pig-derived cells, sheep-derived cells, goat-derived cells, dog-derived cells, cat-derived cells or rodents-derived cells are preferable; human-derived cells or non-human primates-derived cells are more preferable; and human-derived cells are further more preferable. Additionally, although the cells comprising the cell population of the present invention may be cells derived from any tissues, cells derived from bone marrow, cells derived from spleen, cells derived from cord blood, cells derived from peripheral blood, cells derived from fat tissue or cells derived from neural tissue are preferable; cells derived from bone marrow, cells derived from cord blood, cells derived from peripheral blood or cells derived from fat tissue is more preferable; cells derived from bone marrow or cells derived from cord blood are further more preferable.

Examples of the stem cells preferably include hematopoietic stem cells, mesenchymal stem cells and neural stem cells. Examples of the hematopoietic stem cells include CD 34-positive hematopoietic stem cells, CD 34-positive CD 38-negative hematopoietic stem cells, CD 34-negative Lineage-negative hematopoietic stem cells and the like. Examples of neural stem cells include CD 34-positive neural stem cells, CD 133-positive neural stem cells, Nestin-positive neural stem cells, Sox2-positive neural stem cells, Musashi1-positive neural stem cells and the like.

Examples of the progenitor cells include hematopoietic progenitor cells, neural progenitor cells and the like. Examples of the hematopoietic progenitor cells include CD 34-positive hematopoietic progenitor cells, CD 34-negative Lineage-negative hematopoietic progenitor cells and the like. Examples of neural progenitor cells include CD-34 positive neural progenitor cells, CD 133-positive neural progenitor cells, Nestin-positive neural progenitor cells, Sox2-positive neural progenitor cells, Musashi1-positive neural progenitor cells and the like.

After recovering these cells from animal individuals, they can also be proliferated by culturing. Additionally, a cell population just after recovery may be used, or the cells after preservation treatment such as freezing or the like or a cell population after a cell fractionation treatment or an enzyme treatment may be used.

The cells of the present invention are excellent in their ability to bind to E-selectin, ability to migrate to the living body tissues and ability to engraft to the living body tissues, so that they are useful for the hematopoietic stem cell transplantation and restoration of damaged tissues which use stem cell transplantation.

Examples of the living body tissues to which the cells of the present invention migrate and engraft include bone marrow tissues or damaged tissues. Examples of the bone marrow tissues include a bone marrow tissue of a patient who underwent a chemotherapy or radiation irradiation. Examples of the damaged tissues include damaged tissues of brain, heart, lung, intestinal tract, kidney, pancreas, liver, skin, blood vessel and the like tissues.

### 2. Preparation method of the cells of the present invention

As the method for preparing the cell population to be used in the present invention, it may be any method which can isolate a cell population in which sialyl Lewis X sugar chain is expressed but Lewis X sugar chain is not substantially expressed. Examples of a specific method include a preparation method by a cell fractionation treatment, a method for treating with α 1,3-fucosyltransferase in the presence of a fucose donor and the like.

Examples of the preparation method by a cell fractionation treatment include a method in which desired cells are recovered by distinguishing sialyl Lewis X sugar chain from Lewis X sugar chain and the like. Examples of said method include a method which uses antibodies capable of recognizing sialyl Lewis X sugar chain and Lewis X sugar chain, a method which uses microbeads and a magnet, a method which uses a column prepared with a carrier to which an antibody is immobilized and the like.

Examples of the method which uses antibodies capable of recognizing sialyl Lewis X sugar chain and Lewis X sugar chain include a method in which the antibodies capable of recognizing respective sugar chains are allowed to react with cells and then a cell population showing high expression of sialyl Lewis X sugar chain and low expression of Lewis X sugar chain is recovered by a cell sorter. The cell population having high expression means a cell population which shows higher fluorescence intensity than that of a negative control cell population. Examples of the negative control include a cell population reacted using an isotype control antibody. The cell population having low expression means a cell population which shows lower fluorescence intensity than that of a positive control. In the case of blood cells for example, examples of the positive control include a cell population reacted with an anti-CD 45 antibody.

Examples of the method which uses microbeads and a magnet include a method in which Lewis X sugar chain-positive cell fraction is removed and then sialyl Lewis X sugar chain-positive cell fraction is recovered or a method in which sialyl Lewis X sugar chain-positive cell fraction is recovered and then Lewis X sugar chain-negative cell fraction is recovered, and the like.

Examples of the method for treating with α 1,3-fucosyltransferase in the presence of a fucose donor include a method in which α 1,3-fucosyltransferase is added to cells in the presence of a fucose donor, a method in which cells are treated in a column, dish, bag or injection syringe which keeps α 1,3-fucosyltransferase and the like. Additionally, cells may be treated using a cell line transferred with a gene encoding for α 1,3-fucosyltransferase or an extract thereof.

As the α 1,3-fucosyltransferase, α 1,3-fucosyltransferase 7 is preferable. Examples of the human α 1,3-fucosyltransferase 7 include a polypeptide consisting of the amino acid sequence of SEQ ID NO:1. Also, the α 1,3-fucosyltransferase may be a polypeptide consisting of an amino acid sequence having a 60% or more of homology with the polypeptide consisting of the amino acid sequence of SEQ ID NO: and also having the α 1,3-fucosyltransferase activity, a polypeptide consisting of an amino acid sequence in which at least one amino acid in the amino acid sequence of SEX ID NO: 1 is substituted, deleted or added and also having the α 1,3-fucosyltransferase activity, a polypeptide encoded by a DNA consisting of the nucleotide sequence of SEQ ID NO:2, or a polypeptide encoded by a DNA which hybridizes with a DNA consisting of a nucleotide sequence complementary to the nucleotide sequence of SEQ ID NO:2 under a stringent condition and also having the α 1,3-fucosyltransferase activity.

Additionally, the gene to be used when cells are treated using a cell line transferred with a gene encoding for α 1,3-fucosyltransferase or an extract thereof may be a gene encoding for a human α 1,3-fucosyltransferase 7, a gene coding for a polypeptide consisting of an amino acid sequence having a 60% or more of homology with the polypeptide consisting of the amino acid sequence of SEQ ID NO: and also having the α 1,3-fucosyltransferase activity, a gene encoding for a polypeptide consisting of an amino acid sequence in which at least one amino acid in the amino acid sequence of SEQ ID NO:1 is substituted, deleted or added and also having the α 1,3-fucosyltransferase activity, or a gene which is a DNA that hybridizes with a DNA consisting of a nucleotide sequence complementary to the nucleotide sequence of SEQ ID NO:2 under a stringent condition and also encodes for a polypeptide having the α 1,3-fucosyltransferase activity.

According to the present invention, the polypeptide having a 60% or more of homology with the amino acid sequence of SEQ ID NO: and also having the α 1,3-fucosyltransferase activity means a polypeptide having at least 60% or more, preferably 70% or more, more preferably 80% or more, further preferably 90% or more, particularly preferably 95% or more, the most preferably 97% or more, of homology with a protein consisting of the amino acid sequence of SEQ ID NO:1, when calculated using analyzing software such as BLAST [J. Mol. Biol., 215, 403 (1990)], FASTA [Methods in Enzymology, 183, 63 (1990) ] and the like.

According to the present invention, examples of the protein consisting of an amino acid sequence in which at least one amino acid in the amino acid sequence of SEQ ID NO:1 is deleted, substituted, inserted and/or added and also having the α 1,3-fucosyltransferase activity include a protein which can be obtained by introducing a site-directed mutation into a DNA encoding for a protein having the amino acid sequence of SEQ ID NO:1 using the site-directed mutagenesis described, for example, in Molecular Cloning, 2nd edition, Current Protocols in Molecular Biology, Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985) and the like. Although the number of amino acids to be deleted, substituted, inserted and/or added is one or more and the number is not particularly limited, it is such a degree of number that they can be deleted, substituted or added by conventionally known techniques such as the above-mentioned site-directed mutagenesis and the like. For example, it is from 1 to scores, preferably from 1 to 20, more preferably from 1 to 10, further preferably from 1 to 5.

According to the present invention, the DNA which hybridizes under a stringent condition means a DNA such as a DNA having the nucleotide sequence of the SEQ ID NO:2, or a DNA which can be obtained using a partial fragment thereof as the probe by colony hybridization, plaque hybridization, Southern blot hybridization or the like. Its specific example is a DNA which can be identified by carrying out a hybridization using a filter to which colony-or plaque-derived DNA molecules have been immobilized, at 65°C in the presence of from 0.7 to 1.0 mol/l of sodium chloride, and then washing the filter at 65°C with from 0.1 to 2 times concentration of SSC solution (composition of 1 × SSC solution consists of 150 mmol/l sodium chloride and 15 mmol/l sodium citrate). The hybridization can be carried out in accordance with the methods described in Molecular Cloning, 2nd edition, Current Protocols in Molecular Biology, DNA Cloning 1: Core Techniques, A Practical Approach, Second Edition, Oxford University (1995) and the like. Examples of hybridizable DNA include a DNA having at least 60% or more of homology with the nucleotide sequence of SEQ ID NO:2, or a DNA having preferably 70% or more, more preferably 80% or more, further preferably 90% or more, particularly preferably 95% or more, the most preferably 97% or more, of the homology.

A donor of fucose may be any donor as long as it can donate fucose to α1,3-fucosyltransferase. Examples thereof include GDP-fucose and the like.

### 3. Method for examining migration ability and engraftment ability of the cells of the present invention to living body tissues

Examples of the method for examining migration ability and engraftment ability of the cell population of the present invention to living body tissues include a method in which the presence of human hematopoietic stem cells transplanted into a mouse in the mouse bone marrow is examined. Cells in the mouse bone marrow can be examined by transplanting the human hematopoietic stem cells by injecting through the caudal vein after irradiating a semi-lethal dose of X rays to the mouse, and recovering bone marrow cells from thighbone and shinbone. For example, the ability to migrate to the mouse bone marrow can be examined by analyzing the number of cells of the human CD 45-positive and human CD 34-positive cells, and the ability to engraft to the mouse bone marrow can be examined by calculating the ratio of the human CD 45-positive cell occupying the total number of human CD 45-positive cells and mouse CD-45 positive cells.

Also the methods include a method for examining the presence of the human mesenchymal stem cell in mouse tissues, transplanted in the same manner into mice. For example, cells in a tissue can be examined by transplanting the human mesenchymal stem cell into a carbon tetrachloride-induced hepatopathy or pulmonary disorder [BioFactors, 26, 81 - 92 (2006)] model animal through a caudal vein or other vein, or by directly administering to the tissue. For example, since the human mesenchymal stem cell is CD90-positive and hepatic cells, pulmonary cells or the like bind to streptoavidin, the ability of the human mesenchymal stem cell to migrate to each tissue can be examined by calculating the ratio of CD90-positive cells per streptoavidin binding cells.

Also the methods include a method for examining the presence of mouse neural stem cell in mouse tissues, transplanted in the same manner into mice. For example, cells in a tissue can be examined by transplanting a fluorescence-labeled mouse neural stem cell into an animal through a caudal vein or other vein, or by directly administering to the tissue. As the fluorescence-labeled cell, a cell separated from a transgenic mouse [FEBS Lett., 407, 313 - 319 (1997)] which expresses a fluorescent protein Green Fluorescent Protein or a cell prepared by a living cell labeling method [Eur. J. Immunol., 28, 3066 - 3074 (1998)] which uses PKH or the like can be used.

### 4. Pharmaceutical preparation comprising the cells of the present invention

Since the cell population of the present invention is excellent in its binding ability to E-selectin, migration ability to the living body tissues and engraftment ability to living body tissues, a pharmaceutical preparation comprising the cells of the present invention is useful for hematopoietic stem cell transplantation and restoration of damaged tissues using stem cell transplantation.

As the active ingredient, the pharmaceutical preparation comprising the cell population of the present invention can contain the cells of the present invention alone, or together with a pharmacologically acceptable salt thereof or as a mixture with an active ingredient for other optional treatment. Additionally, such a pharmaceutical preparation is produced by an optional method well known in the field of cell pharmaceuticals, by mixing the cell population of the present invention together with one or more pharmacologically acceptable carriers.

It is preferable to use most effective route of administration in carrying out the treatment, and examples thereof include intravenous or the like route regarding the pharmaceutical preparation comprising the cell population of the present invention.

A pharmaceutical preparation which is suitable for injections and the like consists of a sterile aqueous preparation comprising an active compound preferably isotonic with the acceptor's blood. In the case of injections for example, a solution for injection can be prepared using a carrier or the like consisting of a salt solution, a glucose solution or a mixture of saline and a glucose solution as occasion demands. Additionally, one or more auxiliary components selected from diluents, antiseptics, flavors, fillers, disintegrators, lubricants, binders, surfactants, plasticizers and the like can also be added according to the necessity.

Although dose and administration frequency of the pharmaceutical preparation comprising the cell population of the present invention or a pharmacologically acceptable salt thereof vary depending on administration form, age and weight of each patient and the property or seriousness of the symptom to be treated, it is preferble to administer cells in the number of 100 or more per once.

### 5. Transplantation method of the cell of the invention

The transplantation method of the cell of the present invention may be any method as long as it is a method transplantable to human. Specifically, although the method include a method by drip infusion or intravenous injection, it may be a method for directly administering to various tissues such as bone marrow, brain, heart, lung, intestinal tract, kidney, pancreas, liver and the like.

Although the following describes the present invention based on Examples, the following examples are disclosed only for the purpose of illustration. Accordingly, the scope of the present invention is not limited to the following Examples.

### [Example 1]

### Preparation of antibodies, selectin-IgG fusion proteins and α 1,3-fucosyltransferases

A fluorescein isothiocyanate (FITC)-labeled anti-human Lewis X antibody (HI 98), an FITC-labeled anti-mouse IgM antibody (II/41), an IgM isotype control antibody (11E10), a phycoerythrin (PE)-labeled anti-human CD38 antibody (HIT 2), a PE-labeled anti-mouse CD45 antibody (30-F11), a PE-labeled anti-mouse Tear-119 antibody (TER-119) and an allophycocyanin (APC)-labeled human CD34 antibody (581) were purchased from Becton Dickinson Pharmingen . FITC-labeled and APC-labeled human CD45 antibodies (HI30) were purchased from eBioscience. An FITC-labeled anti-human IgG antibody was purchased from DAKOCytomation.

As the anti-human sialyl Lewis X sugar chain antibody (KM 93), an antibody collected from a hybridoma culture supernatant was used [Hanai et al., Cancer Research, 46, 4438 - 4443 (1986)].

Production of a fusion protein of human E-selectin with human IgG constant region (Fc) (to be referred to as E-selectin-Fc chimeric protein hereinafter) and a fusion protein of human P-selectin with Fc (to be referred to as P-selectin-Fc chimeric protein hereinafter) was carried out in accordance with the conventionally known method [Yago et al., Journal of Immunology, 15, 707 - 714 (1997)]. Additionally, production of a fusion protein of human L-selectin with Fc (to be referred to as L-selectin-Fc chimeric protein hereinafter) [Siegelman et al., Proc. Natl. Acad. Sci., USA 86, 5562 - 5566 (1989)] and a fusion protein of human CD 40 with Fc (to be referred to as CD 40-Fc chimeric protein hereinafter) [Hollenbaugh et al., EMBO J., 11, 4313 - 4321 (1992)] was carried out in the same manner as described in the above. The α 1,3-fucosyltransferases 6 and 7 (FT 6 and FT 7) were expressed in Namalwa cells and purified [Shinoda et al., Journal of Biological Chemistry, 272, 31992 - 31997 (1997)].

### Human cord blood cells and CD34-positive cells

Regarding hematopoietic stem cells derived from the human cord blood, cord blood mononuclear cells were purchased from AllCells. Thawing and washing of the frozen cells were carried out in accordance with the instructions of AllCells.

After quickly thawing out the frozen cells contained in a vial by putting into a 37°C water bath, DMEM (manufactured by Gibco) containing 10% fetal bovine serum (FBS, manufactured by JRH Biosciences) (to be referred to as 10% FBS/DMEM hereinafter) was slowly added until the volume became 20 ml. After centrifugation operation (1.8 k rpm, 8 minutes at room temperature), the supernatant was discarded leaving several ml of the supernatant. The cell washing operation was carried out twice.

In order to recover hematopoietic stem cells from the cord blood mononuclear cells, CD34-positive cells were separated using a CD34 microbeads kit (manufactured by Miltenyi Biotec) and a separation apparatus AutoMACS^{™} (manufactured by Miltenyi Biotec) in accordance with the descriptions attached to the products.

### Immunodeficiency mice

NOD/SCID mice were purchased from Central Institute for Experimental Animal and CLEA Japan and reared in an isolator of an animal rearing facility.

### α 1,3-Fucosyltransferase treatment

From 0.1 to 10 × 10⁶ cells of CD34-positive cells derived from the cord blood obtained in the above were added to and suspended in 100 to 1,000 µl of a fucosyltransferase reaction solution containing 10 mmol/l MnCl₂ (manufactured by Sigma), 1 mmol/l guanosine diphosphate (GDP)-fucose (manufactured by Kyowa Hakko Kogyo), 0.1% Human Serum Albumin (HSA, manufactured by Sigma) and 20 mmol/l N-2-hydroxyethylpiperazine-N-2-ethanesulfonic acid (HEPES, manufactured by Gibco). After adding 16 µg of α1,3-fucosyltransferase 7 (FT 7) or 3 µg of α 1,3-fucosyltransferase 6 (FT 6), or a fucosyltransferase reaction solution to which the enzyme was not added as an enzyme untreated group instead of a fucosyltransferase reaction solution to which the enzyme was added, per 0.1-1 × 10⁶ of the cells, the reaction was carried out at 37°C for 1 hour under a condition of 5% CO₂.

### Antibody staining and flow cytometry analysis

After washing the enzyme-treated cells with a staining solution, which is 5% FBS-containing PBS, cell staining using antibodies was carried out. The IgM isotype control antibody was used as a negative control, and FITC-labeled anti-human CD45 antibody was used as a positive control. In the expression quantity analysis of sialyl Lewis X sugar chains on the cell surface, an anti-human sialyl Lewis X sugar chain antibody KM 93 was used as the primary antibody, and an FITC-labeled anti-mouse IgM antibody was used as the secondary antibody. In the expression quantity analysis of Lewis X sugar chains on the cell surface, an FITC-labeled anti-human Lewis X sugar chain antibody was used. Also, in order to detect a CD34-positive and CD38-negative cells as human hematopoietic stem cells, antibody staining of cells was carried out using the PE-labeled anti-human CD38 antibody and APC-labeled anti-human CD34 antibody. In order to respectively detect mouse and human blood cells in the transplanted mice, staining was carried out using the FITC-labeled anti-human CD45 antibody and PE-labeled anti-mouse CD45 antibody. In addition, in case where chimera ratio is low, in order to remove erythrocytes, the staining was carried out using the FITC-labeled anti-mouse CD45 antibody, PE-labeled anti-mouse TER-119 antibody and APC-labeled anti-human CD45 antibody.

In order to remove dead cells after the cell washing by a flow cytometry (FACS) analysis, propidium iodide (manufactured by Invitrogen) was added to the cell suspension to a final concentration to be 1 µg/ml. Using a flow cytometry device FACSAria (manufactured by Becton Dickinson), the expression quantity of sialyl Lewis X sugar chains, Lewis X sugar chains and CD45 in a CD34-positive and CD38-negative cell population among the propidium iodide-negative cells as viable cells was analyzed.

### Selectin binding assam

Cells were suspended in D-PBS(+) (manufactured by Gibco) containing 0.5% HSA and 0.1 g/l CaCl₂ (to be referred to as 0.5% HSA%/D-PBS(+) hereinafter), and 1 µg of the E-selectin-Fc chimeric protein, 2 µg of the P-selectin-Fc chimeric protein, 2 µg of the L-selectin-Fc chimeric protein and, as a negative control, 2.2 µg of the CD 40-Fc chimeric protein were respectively added per 10⁶ of the cells. Staining was carried out using the FITC-labeled anti-human IgG antibody as the secondary antibody. The protein binding and secondary antibody reaction were carried out respectively at 4°C for 30 minutes. After carrying out cell washing with the 0.5% HSA%/D-PBS(+), the staining was carried out under a condition of 4°C and 30 minutes by adding the PE-labeled anti-human CD38 antibody and APC-labeled anti-human CD34 antibody. In order to remove dead cells after the cell washing by an FACS analysis, propidium iodide was added to the cell suspension to a final concentration of 1 µg/ml. By setting a CD34-positive and CD38-negative cell population among the propidium iodide-negative cells as viable cells in the FACS analysis by FACSAria, the selectin protein binding ability was analyzed.

### Bone marrow migration assay of human hematopoietic stem cells in NOD/SCID mice

FT7- or FT6-treated CD34-positive cells derived from cord blood or the enzyme-untreated CD34-positive cells as a negative control were transplanted to NOD/SCID mice. At the time of from 8 to 10 weeks of age after 1 week or more of rearing of the mice for acclimatization, a semi-lethal dose of 400 cGy was irradiated using an X-ray irradiation device MBR-1505R (manufactured by Hitachi Medico). The CD34-positive cells were transplanted by caudal vein injection at a dose of 3 × 10⁵ cells per animal. After 12 to 18 hours of the transplantation, each mouse was euthanized to collect bone marrow cells from the thighbone and shinbone. The bone marrow cells were stained by the FITC-labeled anti-human CD45 antibody, PE-labeled anti-mouse CD45 antibody and APC-labeled anti-human CD34 antibody. After washing the cells, erythrocytes were lysed by adding an ammonium chloride hemolytic agent, and propidium iodide was added thereto to be a final concentration of 1 µg/ml. The human CD45-positive and human CD34-positive cells among the propidium iodide-negative cells as the viable cells were analyzed by the FACS analysis using FACSAria. The human CD45-positive and human CD34-positive cells were analyzed by obtaining the data on 2 × 10⁶ of the viable cells.

### Engraftment Assay of human hematopoietic stem cells-transplanted NOD/SCID mice

FT7- or FT6-treated CD34-positive cells derived from cord blood or the enzyme-untreated CD34-positive cells as a negative control were transplanted to NOD/SCID mice irradiated with a semi-lethal X-ray dose of from 250 to 300 cGy. At the time of from 8 to 10 weeks of age after 1 week or more of rearing of the mice for acclimatization, X-ray irradiation was carried out using an X-ray irradiation device MBR-1505R (manufactured by Hitachi Medico) and then the transplantation was carried out. The CD34-positive cells were transplanted by caudal vein injection at a dose of 2 × 10⁵ cells per animal. Additionally, same treatment was carried out for cord blood mononuclear cells and 1 × 10⁷ cells were transplanted similarly to mice to which X-ray had irradiated. After 8 to 9 weeks of the transplantation, each mouse was euthanized to collect bone marrow cells from the thighbone and shinbone. The bone marrow cells were stained by the FITC-labeled anti-human CD45 antibody and PE-labeled anti-mouse CD45 antibody. In case where chimera ratio is low, in order to detect low frequency human blood cells, they were stained by the FITC-labeled anti-mouse CD45 antibody, PE-labeled anti-mouse TER-119 antibody and APC-labeled anti-human CD45 antibody.

After washing the cells, erythrocytes were lysed by adding an ammonium chloride hemolytic agent, and propidium iodide was added thereto to be a final concentration of 1 µg/ml. The human CD45-positive cells and mouse CD45-positive cells among the propidium iodide-negative cells as the viable cells were analyzed by the FACS analysis using FACSAria. When the mouse TER-119 antibody was used, the human CD45-positive cells and mouse CD45-positive cells among the TER-119-negatives were analyzed. The engraftment ratio of human blood cells was calculated as the ratio (%) of human CD45-positive cells occupying the total of the number of human CD45-positive cells and mouse CD45-positive cells.

### Significance test

All values were shown by average value ± SD. Student's t-test was used in the significance test.

### Expression analysis of sialyl Lewis X sugar chains and Lewis X sugar chains in human CD34-positive cell

Results of the analysis of human CD34-positive cells isolated from cord blood mononuclear cells are shown in Fig. 1. As shown in Fig. 1, the purity was 95 to 99%. In order to separate human hematopoietic stem cells, the CD34-positive CD38-negative cells were gated by the area surrounded by the open square of Fig. 1A (5 to 10%). Results of the analysis of expression quantities of sialyl Lewis X sugar chains and Lewis X sugar chains in the CD34-positive CD38-negative cells are shown in Figs. 2A and 2B. As shown in Figs. 2A and 2B, although sialyl Lewis X sugar chains were expressed in this cell population, the expression of Lewis X sugar chains was low.

Next, expression quantities of sialyl Lewis X sugar chains in FT 6- or FT 7-treated CD34-positive CD38-negative cells were analyzed. The results are shown in Fig. 2C and Fig. 2E, respectively. As shown in Fig. 2C and Fig. 2E, both of the enzymes significantly increased expression quantity of sialyl Lewis X sugar chains. Next, expression quantity of Lewis X sugar chains was analyzed. The results are shown in Fig. 2D and Fig. 2F, respectively. As shown in Fig. 2F, expression quantity of Lewis X sugar chains was not increased in the FT 7-treated CD34-positive CD38-negative cells, while as shown in Fig. 2D, the expression quantity of Lewis X sugar chains was significantly increased in the FT 6-treated CD34-positive CD38-negative cells. Digitized results of the analysis is shown in Table 1.

**[Table 1]**

| | **Isotype control** | **Sialyl Lewis X** | **Lewis X** |
|---|---|---|---|
| **Untreated** | **41** | **19562** | **120** |
| **FT6-treated** | **37** | **52410** | **715** |
| **FT7-treated** | **4 -cry** | **34970** | **95** |

Similar analysis was independently carried out 5 times and the results are shown in Table 2. As shown in the Table 2, the aforementioned results showed reproducibility by the 5 analyses.

**[Table 2]**

| | | | | | N=5 |
|---|---|---|---|---|---|
| | **Isotype control** | **Sialyl Lewis X** | **Lewis X** | **CD 45** | |
| **Untreated** | **48 ± 22** | **28254 ± 10265** | **48 ± 19** | **1067 ± 127** | |
| **FT6-treated** | **51 ± 9** | **44130 ± 7997 *** | **133 ± 35 **** | **962 ± 97** | |
| **FT7-treated** | **45 ± 15** | **40370 ± 5542 *** | **34 ± 9** | **1089 ± 161** | |

### Binding activity of human CD34-positive cells for selectin

Binding of FT 6- or FT 7-treated human CD34-positive cells to E-selectin, P-selectin and L-selectin, respectively, was analyzed. The results are shown in Fig. 3 and Table 3. As shown in Fig. 3 and Table 3, the binding ability of human CD34-positive cells to E-selectin was significantly increased by the FT 7 treatment. Additionally, the binding ability to P-selectin was also increased. The increase of binding ability to E-selectin and P-selectin was the same as the result of the case of FT 6 treatment.

**[Table 3]**

| | **CD40** | **E-selectin** | **P-selectin** | **L-selectin** |
|---|---|---|---|---|
| **Untreated** | **202** | **18506** | **13168** | **5448** |
| **FT 6-treated** | **160** | **146589** | **20204** | **4677** |
| **FT 7-treated** | **140** | **109783** | **22738** | **10496** |

### Bone marrow migration activity of human hematopoietic stem cells in NOD/SCID mice

The hematopoietic stem cells transplanted into peripheral blood by intravenous injection disappear from the peripheral blood within several hours and migrate to the bone marrow tissue and engraft thereto. FT6- or FT7-treated CD34-positive cells derived from cord blood or untreated cells as a negative control were transplanted into NOD/SCID mice, and the human CD45-positive human CD34-positive cells in bone marrow after 12 to 18 hours were detected. The results are shown in Fig. 4 and Table 4. As shown in Fig. 4 and Table 4, the bone marrow migration activity was not increased by the FT 6 treatment, whereas it was significantly increased 3.1 to 3.5 times by the FT 7 treatment. Based on the above, it was revealed that among the two kinds of treatments by fucosyltransferases FT 6 and FT 7, the bone marrow migration ability is not increased by the FT 6 treatment at all, but is significantly increased by the FT 7 treatment.

**[Table 4]**

| The number of human CD45-positive, mouse CD45-negative and human CD34 positive cells | | | | |
|---|---|---|---|---|
| | **Untreated** | **FT7-treated** | ***P*** | **Folds** |
| **Test 1** | **253 ± 62** | **773 ± 133** | **0.00363** | **3.1** |
| **Test 2** | **95 ± 13** | **313 ± 72** | **0.00693** | **3.3** |
| **Test 3** | **167 ± 44** | **590 ± 95** | **0.00223** | **3.5** |
| | | | | |
| | **Untreated** | **FT6-treated** | | |
| **Test 4** | **358 ± 110** | **435 ± 181** | **0.62** | |
| **Test 5** | **113 ± 38** | **100 ± 22** | **0.57** | |

### Engraftment activity of human hematopoietic stem cells in NOD/SCID mice

CD34-positive cells treated by fucosyltransferase were transplanted into NOD/SCID mice, and the ratio of human blood cells in the bone marrow after 8 to 9 weeks was examined. The results are shown in Fig. 5. As shown in Fig. 5, since the FT 6-treated cell transplantation group showed similar results of the enzyme-untreated group, there was no engraftment improving effect. On the other hand, the FT 7-treated transplantation group showed a human-derived cell engraftment ratio of 98 to 99%. It shows that almost all of the mouse blood cells were replaced by the human blood cells.

Similar analysis was independently carried out 4 times and the results are shown in Fig. 6. As shown in Fig. 6, it was revealed that the FT 6 treatment does not have the engraftment ratio improving effect, and the engraftment ratio improving effect by the FT 7 treatment is obtained with high reproducibility and stability. Additionally, the engraftment improving effect by the FT 7 treatment and the absence of engraftment improving effect by the FT 6 treatment were also confirmed by a test in which the CD34-positive cells were transplanted after reducing the number of the cells to be transplanted to be a half number, that is, 1 × 10⁶ cells.

In this connection, significant engraftment improving effect in a short time was confirmed in comparison with untreated cells by enzyme in case where cord blood mononuclear cells from which CD34 positive cells had not separated were transplanted after treatment with FT7. On the other hand, in case where cord blood mononuclear cells treated with FT6 were transplanted, significant engraftment improving effect cannot be confirmed in comparison with untreated cells by enzyme.

### [Example 2]

### Migration of mesenchymal stem cells to the liver in a hepatitis model 1. Expression analysis of Lewis X sugar chain and sialyl Lewis X_ sugar chain in human mesenchymal stem cell

The human mesenchymal stem cells (to be referred to as hMSC hereinafter) were purchased from CAMBREX. The hMSC was cultured in a CO₂ incubator using an MSCGM medium (mfd. by CAMBREX) under a condition of 37°C and 5% CO₂. After washing with phosphate buffered saline (to be referred to as PBS (-) hereinafter), the cells were peeled off by adding TrypLE Select (mfd. by Invitrogen) and treating at 37°C for 2 minutes, and then the reaction was stopped by adding PBS(-) solution containing 5% FBS. The cells were recovered by carrying out centrifugation at 1,000 rpm (rotation per minute) for 5 minutes and washed with PBS(-) and then 1-2×10⁶ of the cells were suspended in 80-100 µl of Hanks' Balanced Salt Solutions (HBSS) reaction liquid [25 mmol/l HEPES (mfd. by Gibco), 10 mmol/lMnCl₂ (mfd. by SIGMA), 0.1% HSA, HBSS(-) (mfd. by Gibco)]. After adding 4-16 µg of FT 7 per 1 × 10⁶ cells, GDP-fucose (mfd. by Kyowa Hakko Kogyo) was adjusted to be a final concentration of 1 mmol/l, and incubation was carried out at 37°C for 1 hour. On the other hand, as the negative control, hMSC was incubated using the HBSS(-) reaction liquid alone at 37°C for 1 hour. After washing cells three times with PBS(-), the cells were suspended in the PBS(-) solution containing 5% FBS and dispensed in 1 × 10⁵ cells portions. An FITC-labeled anti-human CD 15 antibody (mfd. by Becton Dickinson) was added as an anti-Lewis X sugar chain antibody at a concentration of 5% and allowed to undergo the reaction at 4°C for 1 hour. On the other hand, anti-human sialyl Lewis X sugar chain antibody KM93 (mfd. by CALBIOCHEM) was added as an anti-sialyl Lewis X sugar chain antibody at a concentration of 5% and allowed to undergo the reaction at 4°C for 30 minutes. After washing with the PBS(-) solution containing 5% FBS, the cells were recovered by centrifuging at 4,500 rpm for 1 minute and suspended in the PBS(-) solution containing 5% FBS. An FITC-labeled anti-mouse IgG antibody (mfd. by Becton Dickinson) was added as the secondary antibody at a concentration of 5% and allowed to undergo the reaction at 4°C for 30 minutes. The cells treated with anti-human Lewis X sugar chain antibody and the cells treated with the anti-sialyl Lewis X sugar chain antibody and then treated with the secondary antibody were washed three times with the PBS(-) solution containing 5% FBS, and then suspended in 1 ml of the PBS(-) solution containing 5% FBS and analyzed by a flow cytometry (Becton Dickinson; FACS Aria). The results are shown in Fig. 7. As shown in Fig. 7, expression of Lewis X sugar chain was not found and expression of sialyl Lewis X sugar chain was hardly found in hMSC. Additionally, it was found that Lewis X sugar chain was not increased, but sialyl Lewis X sugar chain alone was selectively increased by the FT7 treatment.

Also, the cells were suspended in D-PBS(+) (mfd. by Gibco) containing 0.5% HSA and 0.1 g/l CaCl₂ [to be referred to as 0.5% HSA%/D-PBS(+) hereinafter], and 1 µg of E-selectin-Fc chimeric protein, 2 µg of P-selectin-Fc chimeric protein or 2 µg of L-selectin-Fc chimeric protein per 1 to 5 × 10⁵ cells was respectively added thereto. The FITC-labeled anti-human IgG antibody was used as the secondary antibody to carry out staining. The protein binding and secondary antibody reaction were respectively carried out at 4°C for 30 minutes. After washing the cells with 0.5% HSA%/D-PBS(+), in order to eliminate dead cells by the FACS analysis, propidium iodide was added to the cell suspension to be a final concentration of 1 µg/ml. The selectin protein binding ability of propidium iodide-negative cells as the living cells in the FACS analysis by FACSAria was analyzed. The results are shown in Table 5. As shown in Table 5, increase of mean fluorescence intensity (MFI), namely the E, P, L-selectin binding ability, was confirmed by the FT7 treatment.

**[Table 5]**

| | E-selectin | P-selectin | L-selectin |
|---|---|---|---|
| Untreated hMSC | 564 | 8943 | 7266 |
| FT7 enzyme-treated hMSC | 20545 | 13667 | 15388 |

### 2. Preparation of mouse carbon tetrachloride hepatopathy model

Male BALB/c-nu/nu Slc mice of 5 weeks of age (Japan SLC) were acclimatization-reared for 1 week. Carbon tetrachloride (mfd. by Wako Pure Chemical Industries) was diluted 10 times with olive oil (mfd. by Wako Pure Chemical Industries), and intraperitoneal administration of the carbon tetrachloride to the BALB/c-nu/nu Slc mice was carried out at a ratio of 1 ml/kg to induce of acute hepatopathy.

### 3. Transplantation of hMSC into mouse carbon tetrachloride hepatopathy model and analysis of liver-migrated cells (1)

In the same manner as described in the above, the FT 7 enzyme-treated hMSC and the negative control hMSC were suspended in PBS(-) and adjusted to be 1 × 10⁶ cells/ml. The BALB/c-nu/nu Slc mice which has passed 24 hours after the carbon tetrachloride administration were anesthetized by the intramuscular injection of 40 µl/head of a mixed anesthetic liquid [3:3:10:110 mixture of Dormicum Injection (mfd. by Astellas Pharma), Domitor (mfd. by Meiji Seika), Nembutal (mfd. by Dainippon Sumitomo Pharmaceutical) and physiological saline]. By carrying out abdominal section, the FT 7 enzyme-treated hMSC or the control hMSC was transplanted into the spleen at a dose of 1 × 10⁵ cells/head (n = 5 for each group). After 24 hours of the transplantation, the liver was extracted and finely cut into pieces in a collagenase reaction liquid [0.04% collagenase type IV (mfd. by SIGMA), 0.004% trypsin inhibitor (mfd. by SIGMA), 5 mmol/l CaCl₂ (mfd. by Nakalai Tesque), HBSS(-) (mfd. by Nakalai Tesque)], followed by incubation at 37°C for 2 hours. It was filtered using a 100 µm membrane (mfd. by Becton Dickinson) and washed three times with the PBS(-) containing 5% FBS. After suspension in the same solution, it was dispensed in 1 × 10⁸ cells portions. A biotin-labeled Mouse Lineage Panel (mfd. by Becton Dickinson) was added thereto at a concentration of 2% and allowed to undergo the reaction at 4°C for 1 hour. After washing with the PBS(-) solution containing 5% serum, the cells were recovered by centrifuging at 4,500 rpm for 1 minute and suspended in the PBS(-) solution containing 5% FBS. A PerCP-labeled streptoavidin (mfd. by Becton Dickinson) and a PE-labeled anti-human CD 90 antibody (mfd. by Becton Dickinson) were further added thereto at a concentration of 10% and allowed to undergo the reaction at 4°C for 2 hours. After washing three times with the PBS(-) solution containing 5% FBS, the cells were suspended in 1 ml of the same solution and analyzed by the flow cytometry. Since the blood cells derived from liver are treated with the Mouse Lineage Panel and then treated with the PerCP-labeled streptoavidin and the liver cells bind to streptoavidin, all of the collected cells derived from mouse liver can be detected as PerCP-positive cells. On the other hand, since the mouse liver-derived cells do not react with the human CD 90 antibody, the hMSC can be specifically stained. Therefore, the number of PE-positive cells per the number of PerCP-positive cells can be expressed as the migration ratio (%) of the transplanted hMSC to the liver. The results of analysis by flow cytometry are shown in Fig. 8. As shown in Fig. 8, the hMSC transplanted by the FT 7 enzyme treatment showed improved migration ratio to the liver. This shows that the property to migrate to damaged regions was improved in the cells in which the Lewis X sugar chain was not added and the sialyl Lewis X sugar chain alone was added by the FT 7 enzyme treatment.

### 4. Transplantation of hMSC into mouse carbon tetrachloride hepatopathy model and analysis of liver-migrated cells (2)

The FT7 enzyme-treated hMSC or negative control hMSC as described in the foregoing was suspended in PBS(-) and adjusted to 2.5×10⁶ cells/ml. The FT7 enzyme-treated hMSC or control hMSC was transplanted at a dose of 5×10⁵ cells/head from a caudal vein of BALB/c-nu/nu Slc mouse after 24 hours of the administration of carbon tetrachloride (n = 6 for each group). The liver was extracted after 18 hours of the transplantation, treated as described in the foregoing and then subjected to a hemolysis treatment, and the cells were dispensed in 3×10⁶ portions.

PE-Cy7-labeled streptoavidin (mfd. by Becton Dickinson) and PE-labeled human CD90 antibody were added thereto at a concentration of 10%, and FITC-labeled mouse CD45 antibody (mfd. by Becton Dickinson) and FITC-labeled mouse Ter 119 antibody (mfd. by Becton Dickinson) were added at a concentration of 5%, and allowed to undergo the reaction at 4°C for 2 hours. The cells were washed three times with a PBS(-) solution containing 5% FBS, suspended in 1 ml of the same solution and then analyzed by a flow cytometry. The FITC-negative PE-Cy7-positive cells after removal of the FITC-labeled blood cells were regarded as hepatic cells, and the PE-positive cells were regarded as hMSC, the number of cells of hMSC per one million hepatic cells was calculated. The results are shown in Fig. 9. As shown in Fig. 9, significant increase in the number of cells migrated to the liver was found by the treatment with FT7.

### 5 Transplantation of hMSC into mouse carbon tetrachloride hepatopathy model and analysis of serum biochemical parameters

By the same method of the above item 4, the FT7 enzyme-treated hMSC or negative control hMSC was transplanted into hepatopathy mice or the solvent (PBS) was administered thereto (n = 6 for each group). After 18 hours of the transplantation, blood samples were collected and total bilirubin (T-BIL), aspartate aminotransferase (AST), alanine aminotransferase (ALT), alanine phosphatase (ALP) and lactate dehydrogenase (LDH) in seram were measured by Fuji Dry Chem (mfd. by Fuji Photo Film). The results are shown in Fig. 10. As shown in Fig. 10, significant improvement of the blood biochemical parameters was found by the treatment with FT7.

### [Example 3]

### Migration of mesenchymal stem cell to the lung in a pneumonia model

### 1. Preparation of mouse carbon tetrachloride lung disorder model

Male BALB/c-nu/nu Sic mice of 5 weeks of age (Japan S L C) were acclimatization-reared for 1 week. An acute lung disorder was induced by diluting carbon tetrachloride (mfd. by Wako Pure Chemical Industries) 10 times with olive oil (mfd. by Wako Pure Chemical Industries) and carrying out intraperitoneal administration of the carbon tetrachloride to the BALB/c-nu/nu Sic mice at a dose of 1 ml/kg [Mizuguchi et al., BioFactors, 26, 81 - 92 (2006)].

### 2. Transplantation of hMSC into mouse carbon tetrachloride lung disorder model and analysis

By the same method of Example 2, the FT7 enzyme-treated hMSC or negative control hMSC was suspended in PBS(-) and adjusted to 2.5×10⁶ cells/ml. The FT7 enzyme-treated hMSC or control hMSC was transplanted at a dose of 5×10⁵ cells/head into BALB/c-nu/nu Slc mouse after 24 hours of the administration of carbon tetrachloride (5 animals for each group). The lungs were extracted after 18 hours of the transplantation, cut into pieces in a collagenase-elastase reaction liquid [150 U/ml collagenase type IV (mfd. by SIGMA), 10 U/ml elastase (mfd. by Nakalai Tesque) DMEM (mfd. by Invitrogen)] and then incubated at 37°C for 2 hours. The cells were collected by filtration using a 100 µm membrane (mfd. by Becton Dickinson); subjected to a hemolysis treatment; washed twice with a PBS (-) solution containing 5% FBS and then suspended in the same solution and dispensed in 3×10⁶ portions. The PE-Cy7-labeled streptoavidin and PE-labeled human CD90 antibody were added thereto at a concentration of 10%, and the FITC-labeled anti-mouse CD45 antibody and FITC-labeled anti-mouse Ter 119 antibody were added at a concentration of 5%, and allowed to undergo the reaction at 4°C for 2 hours. The cells were washed three times with the PBS(-) solution containing 5% FBS, suspended in 1 ml of the same solution and then analyzed by a flow cytometry. The FITC-negative PE-Cy7-positive cells after removal of the FITC-labeled blood cells were regarded as lung cells, and the PE-positive cells were regarded as hMSC, the number of cells of hMSC per one hundred thousand lung cells was calculated. The results are shown in Fig. 11. As shown in Fig. 11, significant increase in the number of cells migrated to the lungs was found by the treatment with FT7.

### [Example 4]

### Preparation of antibodies, selectin-IgG fusion proteins and α1,3-fucosyltransferase

Fluorescein isothiocyanate (FITC)-labeled anti-Lewis X antibody (HI98), FITC-labeled anti-mouse IgM antibody (II/41) and IgM isotype control antibody (11E10) were purchased from Becton Dickinson Pharmingen. FITC-labeled or APC-labeled human CD45 antibody (HI30) was purchased from eBioscience. FITC-labeled anti-human IgG antibody was purchased from DAKOCytomation.

As the anti-sialyl Lewis X sugar chain antibody (KM 93), the antibody recovered from a hybridoma culture supernatant was used [Hanai et al., Cancer Research, 46, 4438 - 4443 (1986)].

Production of a fusion protein of human E-selectin with human IgG constant region (Fc) (to be referred to as E-selectin-Fc chimeric protein) and a fusion protein of human P-selectin with Fc (to be referred to as P-selectin-Fc chimeric protein) was carried out in accordance with the conventionally known method [Yago et al., Journal of Immunology, 15, 707-714 (1997)]. Additionally, production of the fusion protein of human L-selectin with Fc (to be referred to as L-selectin-Fc chimeric protein) [Siegelman et al., Proc. Nat. Acad. Sci. USA, 86, 5562-5566 (1989)] and the fusion protein of human CD40 with Fc (to be referred to as CD40-Fc chimeric protein) [Hollenbaugh et al., EMBO J., 11, 4313-4321 (1992)] was carried out in the same manner as in the above. The α1,3-fucosyltransferase 7 (FT7) was expressed by Namalwa cell and purified [Shinoda et al., Journal of Biological Chemistry, 272, 31992 - 31997 (1997)].

### Culturing of neural stem cell

Neurosphere is a cell containing neural stem cell and neural progenitor cell. Using a frozen neurosphere derived from a mouse fetal central nervous system (Cortex) (mfd. by Stem Cell Technologies), thawing, washing and culturing of the frozen cells were carried out in accordance with the instructions of Stem Cell Technologies. After thawing of the frozen cells, the cells were washed using NeuroCult proliferation medium (mfd. by Stem Cell Technologies) and by centrifugation, suspended in NeuroCult proliferation medium supplemented with 20 ng/ml of recombinant human epidermal growth factor (rh EGF, mfd. by Stem Cell Technologies) and cultured at 37°C for 7 days in a 5% CO₂ incubator. The thus proliferated neurosphere was recovered, and the neurosphere was separated into single cells and suspended using NeuroCult Chemical dissociation Kit (mfd. by Stem Cell Technologies).

### α1,3 -Fucosyltransferase treatment

From 0.1 to 10×10⁵ of the neurosphere-derived cells obtained in the above and suspended into single cells were added into a fucosyltransferase reaction solution containing 10 mmol/l MnCl₂ (mfd. by SIGMA), 1 mmol/l guanosine diphosphate (GDP)-fructose (mfd. by Kyowa Hakko Kogyo), 0.1% Human Serum Albumin (HSA, mfd. by SIGMA) and 20 mmol/l N-2-hydroxyethylpiperazine-N-2-ethanesulfonic acid (HEPES, mfd. by Gibco) to make from 100 to 1,000 µl of suspension. Using a reaction solution to which 16 µg of α1,3-fucosyltransferase 7 (FT7) per 0.1 to 1×10⁵ cells was added, or a fucosyltransferase reaction solution to which the enzyme was not added as an enzyme-untreated group instead of the enzyme-added fucosyltransferase reaction solution, the cells were allowed to undergo the reaction for 1 hour under conditions of 37°C and 5% CO₂.

### Antibody staining and flow cytometry analysis

After washing the enzyme-treated cells with a staining solution, 5% FBS-containing PBS, cell staining was carried out using antibodies. As a negative control, an IgM isotype control antibody was used. In the analysis of expression quantity of the sialyl Lewis X sugar chain on the cell surface, an anti-human sialyl Lewis X sugar chain antibody KM 93 was used as the primary antibody, and an FITC-labeled anti-mouse IgM antibody was used as the secondary antibody. An FITC-labeled anti-Lewis X sugar chain antibody was used in the analysis of expression quantity of the Lewis X sugar chain on the cell surface.

In order to remove dead cells by the flow cytometry (FACS) analysis after the cell washing, propidium iodide (mfd. by Invitrogen) was added to the cell suspension to a final concentration of 1 µg/ml. The expression quantities of sialyl Lewis X sugar chain and Lewis X sugar chain of the living cells, propidium iodide-negative cells, were analyzed using a flow cytometry instrument FACSAria (mfd. by Becton Dickinson).

### Selectin binding assay

The cells were suspended in D-PBS(+) (mfd. by Gibco) containing 0.5% HSA and 0.1 g/l CaCl₂ (to be referred to as 0.5% HSA%/D-PBS(+) hereinafter), and 1 µg of E-selectin-Fc chimeric protein, 2 µg of P-selectin-Fc chimeric protein, 2 µg of L-selectin-Fc chimeric protein or 2.2 µg of a negative control CD40-Fc chimeric protein per 10⁶ cells was respectively added thereto. The FITC-labeled anti-human IgG antibody was used as the secondary antibody to carry out staining. The protein binding and secondary antibody reaction were respectively carried out at 4°C for 30 minutes. After washing the cells with 0.5% HSA%/D-PBS(+), in order to remove dead cells by the FACS analysis, propidium iodide was added to the cell suspension to a final concentration to be 1 µg/ml. The selectin protein binding ability of propidium iodide-negative cells as the living cells in the FACS analysis by FACSAria was analyzed.

### Expression analysis of sialyl Lewis X sugar chain and Lewis X sugar chain of mouse neurosphere

A result of the analysis of expression quantities of sialyl Lewis X sugar chain and Lewis X sugar chain of mouse neurosphere is shown in Fig. 12. As shown in the upper row (untreated group) of Fig. 12, mouse neurosphere was negative in the expression of sialyl Lewis X sugar chain and Lewis X sugar chain.

Next, expression quantities of sialyl Lewis X sugar chain and Lewis X sugar chain of FT7-treated mouse neurosphere were analyzed.

The results are shown in the lower row (FT7 treatment) of Fig. 12. As shown in the lower row of Fig. 12, FT7 treatment significantly increased expression quantity of sialyl Lewis X sugar chain. On the other hand, there was no change in the expression quantity of Lewis X sugar chain.

### Binding activity of neural stem cell for selectin

Binding of FT7-treated mouse neurosphere-derived neural stem cell or neural progenitor cell for each of P-selectin, E-selectin and L-selectin was analyzed. The results are shown in Fig. 13. As shown in Fig. 13, binding ability of the mouse neurosphere-derived neural stem cell or neural progenitor cell for E-selectin was significantly increased by the FT7 treatment.

### Industrial Applicability

According to the present invention, a cell population having excellent migration ability and engraftment ability to tissues, a pharmaceutical preparation comprising said cell population and a transplantation method of said cell population are provided. The cell population of the present invention is useful for restoration of damaged tissues and the like.

## Claims

1. An isolated cell population, wherein a sialyl Lewis X sugar chain is expressed and a Lewis X sugar chain is not substantially expressed.

2. The cell population according to claim 1, which is obtained by a cell fractionation treatment.

3. The cell population according to claim 1 or 2, which is obtained by treating a cell with an α 1,3-fucosyltransferase in the presence of a fucose donor.

4. The cell population according to claim 3, wherein the α 1,3-fucosyltransferase is α 1,3-fucosyltransferase 7.

5. The cell population according to claim 3, wherein the α 1,3-fucosyltransferase is a polypeptide consisting of the amino acid sequence of SEQ ID NO:1.

6. The cell population according to claim 3, wherein the α 1,3-fucosyltransferase consists of an amino acid sequence having a homology of 60% or more with a polypeptide consisting of the amino acid sequence of SEQ ID NO1l and also has α 1,3-fucosyltransferase activity.

7. The cell population according to claim 3, wherein the α 1,3-fucosyltransferase consists of an amino acid sequence in which at least one amino acid in the amino acid sequence of SEQ ID NO: is substituted, deleted or added, and also has α 1,3-fucosyltransferase activity.

8. The cell population according to claim 3, wherein the α 1,3-fucosyltransferase is a polypeptide encoded by a DNA consisting of the nucleotide sequence of SEQ ID NO:2.

9. The cell population according to claim 3, wherein the α 1,3-fucosyltransferase is a polypeptide encoded by a DNA which hybridizes with a DNA consisting of a nucleotide sequence complementary to the nucleotide sequence of SEQ ID NO:2 under a stringent condition, and also is a polypeptide having α 1,3-fucosyltransferase activity.

10. The cell population according to any one of the claims 1 to 9, wherein the cell is a stem cell or a progenitor cell.

11. The cell population according to claim 10, wherein the stem cell or progenitor cell is a hematopoietic stem cell or a hematopoietic progenitor cell.

12. The cell population according to claim 11, wherein the hematopoietic stem cell or hematopoietic progenitor cell is a CD 34-positive hematopoietic stem cell or a CD 34-positive hematopoietic progenitor cell.

13. The cell population according to claim 12, wherein the CD 34-positive hematopoietic stem cell is a CD 38-negative hematopoietic stem cell.

14. The cell population according to claim 11, wherein the hematopoietic stem cell or hematopoietic progenitor cell is a CD 34-negative and Lineage-negative hematopoietic stem cell or CD 34-negative and Lineage-negative hematopoietic progenitor cell.

15. The cell population according to claim 10, wherein the stem cell is a mesenchymal stem cell.

16. The cell population according to claim 10, wherein the stem cell or progenitor cell is a neural stem cell or neural progenitor cell.

17. The cell population according to claim 16, wherein the neural stem cell or neural progenitor cell is Nestin-positive neural stem cell or Nestin-positive neural progenitor cell.

18. The cell population according to claim 16, wherein the neural stem cell or neural progenitor cell is Sox2-positive neural stem cell or Sox2-positive neural progenitor cell.

19. The cell population according to claim 16, wherein the neural stem cell or neural progenitor cell is Musashi1-positive neural stem cell or Musashi 1-positive neural progenitor cell.

20. The cell population according to claim 16, wherein the neural stem cell or neural progenitor cell is CD34-positive neural stem cell or CD34-positive neural progenitor cell.

21. The cell population according to claim 16, wherein the neural stem cell or neural progenitor cell is CD133-positive neural stem cell or CD133-positive neural progenitor cell.

22. The cell population according to any one of the claims 1 to 21, wherein the cell is a human-derived cell.

23. The cell population according to any one of the claims 1 to 22, wherein the cell is a cell derived from a tissue selected from the group consisting of bone marrow, spleen, cord blood, peripheral blood, fat tissue and neural tissue.

24. A pharmaceutical preparation, which comprises the cell population according to any one of the claims 1 to 23.

25. A method for transplanting a cell population, which comprises administering the cell population according to any one of the claims 1 to 23 to a living body.

26. A method for producing a cell expressing sialyl Lewis X sugar chain but not substantially expressing Lewis X sugar chain, which comprises obtaining the cell by a fractionation treatment.

27. A method for producing a cell expressing sialyl Lewis X sugar chain but not substantially expressing Lewis X sugar chain, which comprises obtaining the cell by treating with an α 1,3-fucosyltransferase in the presence of a fucose donor.

28. The production method according to claim 27, wherein the α 1,3-fucosyltransferase is α 1,3-fucosyltransferase 7.

29. The production method according to claim 27, wherein the α 1,3-fucosyltransferase is a polypeptide consisting of the amino acid sequence of SEQ ID NO: 1.

30. The production method according to claim 27, wherein the α 1,3-fucosyltransferase is a polypeptide consisting of an amino acid sequence having a 60% or more of homology with the polypeptide consisting of the amino acid sequence of SEQ ID NO: and also having the α 1,3-fucosyltransferase activity.

31. The production method according to claim 27, wherein the α 1,3-fucosyltransferase is a polypeptide consisting of an amino acid sequence in which at least one amino acid in the amino acid sequence of SEQ ID NO: is substituted, deleted or added and also having the α 1,3-fucosyltransferase activity.

32. The production method according to claim 27, wherein the α 1,3-fucosyltransferase is a polypeptide encoded by a DNA consisting of the nucleotide sequence of SEQ ID NO:2.

33. The production method according to claim 27, wherein the α 1,3-fucosyltransferase is a polypeptide encoded by a DNA which hybridizes with a DNA consisting of a nucleotide sequence complementary to the nucleotide sequence of SEQ ID NO:2 under a stringent condition, and also having the α 1,3-fucosyltransferase activity.

34. The production method according to any one of the claims 26 to 33, wherein the cell is a stem cell or a progenitor cell.

35. The production method according to claim 34, wherein the stem cell or progenitor cell is a hematopoietic stem cell or a hematopoietic progenitor cell.

36. The production method according to claim 35, wherein the hematopoietic stem cell or hematopoietic progenitor cell is a CD 34-positive hematopoietic stem cell or a CD 34-positive hematopoietic progenitor cell.

37. The production method according to claim 36, wherein the CD 34-positive hematopoietic stem cell is a CD 38-negative hematopoietic stem cell.

38. The production method according to claim 35, wherein the hematopoietic stem cell or hematopoietic progenitor cell is a CD 34-negative and Lineage-negative hematopoietic stem cell or CD 34-negative and Lineage-negative hematopoietic progenitor cell.

39. The production method according to claim 34, wherein the stem cell is a mesenchymal stem cell.

40. The production method according to claim 34, wherein the stem cell or progenitor cell is a neural stem cell or neural progenitor cell.

41. The production method according to claim 40, wherein the neural stem cell or neural progenitor cell is Nestin-positive neural stem cell or Nestin-positive neural progenitor cell.

42. The production method according to claim 40, wherein the neural stem cell or neural progenitor cell is Sox2-positive neural stem cell or Sox2-positive neural progenitor cell.

43. The production method according to claim 40, wherein the neural stem cell or neural progenitor cell is Musashi1-positive neural stem cell or Musashi 1-positive neural progenitor cell.

44. The production method according to claim 40, wherein the neural stem cell or neural progenitor cell is CD34-positive neural stem cell or CD34-positive neural progenitor cell.

45. The production method according to claim 40, wherein the neural stem cell or neural progenitor cell is CD133-positive neural stem cell or CD133-positive neural progenitor cell.

46. The production method according to any one of the claims 26 to 45, wherein the cell is a human-derived cell.

47. The production method according to any one of the claims 26 to 46, wherein the cell is a cell derived from a tissue selected from the group consisting of bone marrow, spleen, cord blood, peripheral blood, fat tissue and neural tissue.

48. A pharmaceutical preparation, which comprises a cell obtained by the production method according to any one of the claims 26 to 47.

49. A method for transplanting a cell, which comprises administering a cell obtained by the production method according to any one of the claims 26 to 47 to a living body.


SEQUENCE LISTING

<110>  Kyowa Hakko Kogyo Co., Ltd.

<120>  A cell population with enhanced transplantation activity

<130>  1927

<150>  JP2007-206823
<151>  2007-08-08

<160>  2

<170>  PatentIn version 2.1

<210> 1
<211> 342
<212> PRT
<213> Homo sapiens

<400> 1

Met Asn Asn Ala Gly His Gly Pro Thr Arg Arg Leu Arg Gly Leu Gly
1               5                   10                  15

Val Leu Ala Gly Val Ala Leu Leu Ala Ala Leu Trp Leu Leu Trp Leu
            20                  25                  30

Leu Gly Ser Ala Pro Arg Gly Thr Pro Ala Pro Gln Pro Thr Ile Thr
        35                  40                  45

Ile Leu Val Trp His Trp Pro Phe Thr Asp Gln Pro Pro Glu Leu Pro
    50                  55                  60

Ser Asp Thr Cys Thr Arg Tyr Gly Ile Ala Arg Cys His Leu Ser Ala
65                  70                  75                  80

Asn Arg Ser Leu Leu Ala Ser Ala Asp Ala Val Val Phe His His Arg
                85                  90                  95

Glu Leu Gln Thr Arg Arg Ser His Leu Pro Leu Ala Gln Arg Pro Arg
            100                 105                 110

Gly Gln Pro Trp Val Trp Ala Ser Met Glu Ser Pro Ser His Thr His
        115                 120                 125

Gly Leu Ser His Leu Arg Gly Ile Phe Asn Trp Val Leu Ser Tyr Arg
    130                 135                 140

Arg Asp Ser Asp Ile Phe Val Pro Tyr Gly Arg Leu Glu Pro His Trp
145                 150                 155                 160

Gly Pro Ser Pro Pro Leu Pro Ala Lys Ser Arg Val Ala Ala Trp Val
                165                 170                 175

Val Ser Asn Phe Gln Glu Arg Gln Leu Arg Ala Arg Leu Tyr Arg Gln
            180                 185                 190

Leu Ala Pro His Leu Arg Val Asp Val Phe Gly Arg Ala Asn Gly Arg
        195                 200                 205

Pro Leu Cys Ala Ser Cys Leu Val Pro Thr Val Ala Gln Tyr Arg Phe
    210                 215                 220

Tyr Leu Ser Phe Glu Asn Ser Gln His Arg Asp Tyr Ile Thr Glu Lys
225                 230                 235                 240

Phe Trp Arg Asn Ala Leu Val Ala Gly Thr Val Pro Val Val Leu Gly
                245                 250                 255

Pro Pro Arg Ala Thr Tyr Glu Ala Phe Val Pro Ala Asp Ala Phe Val
            260                 265                 270

His Val Asp Asp Phe Gly Ser Ala Arg Glu Leu Ala Ala Phe Leu Thr
        275                 280                 285

Gly Met Asn Glu Ser Arg Tyr Gln Arg Phe Phe Ala Trp Arg Asp Arg
    290                 295                 300

Leu Arg Val Arg Leu Phe Thr Asp Trp Arg Glu Arg Phe Cys Ala Ile
305                 310                 315                 320

Cys Asp Arg Tyr Pro His Leu Pro Arg Ser Gln Val Tyr Glu Asp Leu
                325                 330                 335

Glu Gly Trp Phe Gln Ala
            340


<210> 2
<211> 1029
<212> DNA
<213> Homo sapiens

<220>
<221> CDS
<222> (1)..(1029)

<400> 2

atg aat aat gct ggg cac ggc ccc acc cgg agg ctg cga ggc ttg ggg     48
Met Asn Asn Ala Gly His Gly Pro Thr Arg Arg Leu Arg Gly Leu Gly
  1               5                  10                  15

gtc ctg gcc ggg gtg gct ctg ctc gct gcc ctc tgg ctc ctg tgg ctg     96
Val Leu Ala Gly Val Ala Leu Leu Ala Ala Leu Trp Leu Leu Trp Leu
             20                  25                  30

ctg ggg tca gcc cct cgg ggt acc ccg gca ccc cag ccc acg atc acc    144
Leu Gly Ser Ala Pro Arg Gly Thr Pro Ala Pro Gln Pro Thr Ile Thr
         35                  40                  45

atc ctt gtc tgg cac tgg ccc ttc act gac cag ccc cca gag ctg ccc    192
Ile Leu Val Trp His Trp Pro Phe Thr Asp Gln Pro Pro Glu Leu Pro
     50                  55                  60

agc gac acc tgc acc cgc tac ggc atc gcc cgc tgc cac ctg agt gcc    240
Ser Asp Thr Cys Thr Arg Tyr Gly Ile Ala Arg Cys His Leu Ser Ala
 65                  70                  75                  80

aac cga agc ctg ctg gcc agc gcc gac gcc gtg gtc ttc cac cac cgc    288
Asn Arg Ser Leu Leu Ala Ser Ala Asp Ala Val Val Phe His His Arg

85                  90                  95

gag ctg cag acc cgg cgg tcc cac ctg ccc ctg gcc cag cgg ccg cga     336
Glu Leu Gln Thr Arg Arg Ser His Leu Pro Leu Ala Gln Arg Pro Arg
            100                 105                 110

ggg cag ccc tgg gtg tgg gcc tcc atg gag tct cct agc cac acc cac     384
Gly Gln Pro Trp Val Trp Ala Ser Met Glu Ser Pro Ser His Thr His
        115                 120                 125

ggc ctc agc cac ctc cga ggc atc ttc aac tgg gtg ctg agc tac cgg     432
Gly Leu Ser His Leu Arg Gly Ile Phe Asn Trp Val Leu Ser Tyr Arg
    130                 135                 140

cgc gac tcg gac atc ttt gtg ccc tat ggc cgc ctg gag ccc cac tgg     480
Arg Asp Ser Asp Ile Phe Val Pro Tyr Gly Arg Leu Glu Pro His Trp
145                 150                 155                 160

ggg ccc tcg cca ccg ctg cca gcc aag agc agg gtg gcc gcc tgg gtg     528
Gly Pro Ser Pro Pro Leu Pro Ala Lys Ser Arg Val Ala Ala Trp Val
                165                 170                 175

gtc agc aac ttc cag gag cgg cag ctg cgt gcc agg ctg tac cgg cag     576
Val Ser Asn Phe Gln Glu Arg Gln Leu Arg Ala Arg Leu Tyr Arg Gln
            180                 185                 190

ctg gcg cct cat ctg cgg gtg gat gtc ttt ggc cgt gcc aat gga cgg     624
Leu Ala Pro His Leu Arg Val Asp Val Phe Gly Arg Ala Asn Gly Arg
        195                 200                 205

cca ctg tgc gcc agc tgc ctg gtg ccc acc gtg gcc cag tac cgc ttc     672
Pro Leu Cys Ala Ser Cys Leu Val Pro Thr Val Ala Gln Tyr Arg Phe
    210                 215                 220

tac ctg tcc ttt gag aac tct cag cac cgc gac tac att acg gag aaa     720
Tyr Leu Ser Phe Glu Asn Ser Gln His Arg Asp Tyr Ile Thr Glu Lys
225                 230                 235                 240

ttc tgg cgc aac gca ctg gtg gct ggc act gtg cca gtg gtg ctg ggg     768
Phe Trp Arg Asn Ala Leu Val Ala Gly Thr Val Pro Val Val Leu Gly
                245                 250                 255

ccc cca cgg gcc acc tat gag gcc ttc gtg ccg gct gac gcc ttc gtg     816
Pro Pro Arg Ala Thr Tyr Glu Ala Phe Val Pro Ala Asp Ala Phe Val
            260                 265                 270

cat gtg gat gac ttt ggc tca gcc cga gag ctg gcg gct ttc ctc act     864
His Val Asp Asp Phe Gly Ser Ala Arg Glu Leu Ala Ala Phe Leu Thr
        275                 280                 285

ggc atg aat gag agc cga tac caa cgc ttc ttt gcc tgg cgt gac agg     912
Gly Met Asn Glu Ser Arg Tyr Gln Arg Phe Phe Ala Trp Arg Asp Arg
    290                 295                 300

ctc cgc gtg cga ctg ttc acc gac tgg cgg gaa cgt ttc tgt gcc atc     960
Leu Arg Val Arg Leu Phe Thr Asp Trp Arg Glu Arg Phe Cys Ala Ile
305                 310                 315                 320

tgt gac cgc tac cca cac cta ccc cgc agc caa gtc tat gag gac ctt     1008
Cys Asp Arg Tyr Pro His Leu Pro Arg Ser Gln Val Tyr Glu Asp Leu
                325                 330                 335

gag ggt tgg ttt cag gcc tga 1029
Glu Gly Trp Phe Gln Ala
            340